# EUROPEAN PATENT APPLICATION

(11) **EP 4 219 707 A1**
(43) Date of publication of application: **02.08.2023**
(21) Application number: 21872573.7
(22) Date of filing: 24.09.2021
(51) Int. Cl.: C12N 15/09, C12N 5/0735, C12N 5/074, C12N 5/10

(54) **METHOD FOR PRODUCING EFFECTOR CELL HAVING DESIRED SPECIFICITY**

(30) Priority: 24.09.2020 JP 2020160252
(71) Applicant: Kyoto University, Kyoto-shi, Kyoto 606-8501 (JP); National University Corporation Shiga University of Medical Science, Otsu-shi Shiga 520-2192 (JP)
(72) Inventor: KAWAMOTO, Hiroshi, Kyoto-shi, Kyoto 606-8501 (JP); AGATA, Yasutoshi, Otsu-shi, Shiga 520-2192 (JP); NAGANO, Seiji, Kyoto-shi, Kyoto 606-8501 (JP); TERADA, Koji, Otsu-shi, Shiga 520-2192 (JP); MASUDA, Kyoko, Kyoto-shi, Kyoto 606-8501 (JP); KONDO, Kenta, Otsu-shi, Shiga 520-2192 (JP)
(74) Representative: Boult Wade Tennant LLP
(86) International application number: PCT/JP2021/035150
(87) International publication number: WO 2022/065444

(57) **Abstract**

Provided is a method for producing effector cells expressing a desired specificity determining region. In this method, material cells that have a cassette deck structure with a cassette tape gene comprising a gene encoding a marker protein in their genome, wherein the material cells can be differentiated into the effector cells, and wherein the marker protein can be expressed in the effector cells or progenitor cells thereof when the material cells are differentiated into the effector cells or progenitor cells thereof are provided first. The material cells are proliferated, the proliferated cells are differentiated into the effector cells, and then, the gene encoding the marker protein in the effector cells is exchanged with a gene encoding a protein that contribute the desired specificity.

## Description

### BACKGROUND

### Technical Field:

The present application relates to a method for producing effector cells with a desired specificity. In particular, the present application relates to a method for efficiently producing mature T cells expressing a T cell receptor having a desired specificity or a mature CAR-T cells expressing a chimeric antigen receptor (CAR) having a desired specificity, or their progenitor cells by using Recombinase-mediated Cassette Exchange (RMCE) or genome editing.

### Background Art:

Tumor-infiltrating lymphocytes (TILs) are highly specific T lymphocytes that recognize tumor tissues in the body as foreign substances and infiltrate into tumors. TILs have been reported in 50-80% of patients with various cancers. The presence of TILs are considered evidence that the immune system is responding to cancer. TILs contain multiple types of T lymphocytes with different specificities, and therapeutic effects can be expected if TILs can be amplified in large amounts and administered to the patient. However, it is difficult to extract and proliferate the TILs from each patient, and even if it were possible, it would take time and cost, and so far only limited efficacy has been demonstrated.

Cells obtained by introducing a gene encoding an antigen specific receptor such as TCR or CAR into mature T cells have been proposed for therapy, and CAR-T cells, in particular, have already been approved for clinical use. In order to obtain such cells, mature T cells are randomly introduced with the antigen specific receptor gene by using retrovirus or lentivirus. A more physiological expression pattern can be expected if the TCR or CAR gene is knocked into the original TCR gene locus. In random gene introduction, there are problems that the insertion site cannot be controlled, the risk of damage to the genome, and the difficulty of controlling the expression level. In addition, in order to apply the therapy to simultaneous administration of mature T cells that express various TCRs, like the tumor-infiltrating lymphocytes, it is necessary to introduce TCRs or CARs into mature T cells one by one. That is not realistic.

Some of the present inventors have proposed introducing a TCR gene into pluripotent stem cells, inducing differentiation of the pluripotent stem cells into mature T cells, and using them for cellular immunotherapy (Patent Literatures 1 to 4). Another group has also proposed introducing a CAR gene into pluripotent stem cells (Patent Literature 5). Procedures disclosed in those documents in an enabled manner include the steps of introducing a TCR or CAR genes into iPS cells followed by differentiating the iPS cells into effector cells that exhibit the functions.

The present inventors have also proposed a method of introducing a desired TCR by providing a cassette deck for introducing a TCR gene into the TCR gene locus of ES cells or iPS cells so that the TCR gene can be introduce under the control of the promoter and the endogenous enhancer. (Patent Literature 6). This method utilizes procedures known as Recombinase-mediated Cassette Exchange (RMCE). In this method, a structure like a cassette deck with a cassette tape gene that can be exchanged with an exogenous sequence is placed in the genome of the material cells in advance. The cassette tape gene can be exchanged with another cassette tape gene by using a recombinase such as Cre or Flippase (FLP). Specifically, pluripotent stem cells having the cassette deck structure with an empty cassette tape gene including a drug resistance gene are constructed so that drug resistance gene is expressed under the expression control mechanism of the pluripotent stem cells. Pluripotent stem cells that express exogenous TCR genes under the expression control of the endogenous TCR gene locus can be produced by replacing the empty cassette tape gene with a cassette tape containing a gene encoding the desired TCR. The obtained pluripotent stem cells can be proliferated as necessary, induced to differentiate into mature T cells, and used for cellular immunotherapy.

The method as such has made it possible to efficiently produce cells for cellular therapy that stably express a desired TCR gene or CAR gene. While the procedures to introduce the TCR into mature T cells or T progenitor cells are widely conducted, the procedure to differentiate the pluripotent stem cells into mature T cells is not popular and is not a situation where the procedure is widely used in the clinical field. If the target of a therapy is an antigen that is commonly expressed in many cancers, such as WT1 antigen, a large scale production of mature T cells that have one type of TCR gene targeting the antigen could be useful for the cellular immunotherapy. However, in order to regenerate TILs, it is necessary to isolate the TCRs of the TILs, introduce the TCRs into iPS cells to generate multiple TCR-iPS cells, and then induce differentiation of the TCR-iPS cells into mature T cells. Those procedures are highly labor-intensive and time-consuming.

### CITATION LIST

### PATENT LITERATURE

PTL1: WO2016/010153
PTL2: WO2016/010154
PTL3: WO2016/010155
PTL4: WO2017/179720
PTL 5: WO2014/165707
PTL6: WO2020/022512

### NON PATENT LITERATURE

NPL1: Themeli et al., Nat Biotechnol. (2013) 928-33
NPL 2: Gong Ying et al., Journal of Cell Biology (2015) 1481-1489

### SUMMARY OF INVENTION

### TECHNICAL PROBLEM

An object of the present invention is to provide a method for producing effector cells having a desired specificity-determining site easily. A further object of the present invention is to provide effector cells having a so-called cassette deck structure in the genome that allows easy exchange of specificity-determining sites. Another object of the present invention is to provide a method for simultaneously producing the same type of effector cells having different specificity-determining sites.

### SOLUTION TO PROBLEM

Provided is a method for producing effector cells which expresses a desired protein, comprising the steps of:
(1) providing material cells that has a cassette deck structure with a cassette tape gene containing a gene encoding a marker protein in their genome, wherein the material cells can be differentiated into the effector cells, and wherein the marker protein can be expressed in the effector cells or their progenitor cells when the material cells are differentiated into the effector cells or their progenitor cells;
(2) proliferating the material cells,
(3) differentiating the material cells into the effector cells or progenitor cells thereof; and
(4) replacing the gene encoding the marker protein in the effector cells or progenitor cells thereof with a gene encoding the desired protein.

In order to conduct step (4), RMCE or genome editing can be employed. In one embodiment, RMCE may be employed. In this embodiment, a pair of recombinase target sequences are placed upstream and downstream of the gene encoding the marker protein in step (1). The effector cells or progenitor cells thereof obtained in step (3) will include the gene encoding the marker protein sandwiched by the pair of recombinase target sequences. Thus obtained effector cells or progenitor cells thereof and a cassette tape exchange vector which comprises the same pair of recombinase target sequences and a gene encoding the desired protein between the recombinase target sequences are cultured in the presence of the recombinase so that the gene encoding the marker protein is exchanged with the gene encoding the desired protein.

In another embodiment, the gene encoding the marker protein may be disrupted by means of genome editing and then, introducing the gene encoding the desired protein so that the desired protein is expressed in the material cells.

In another embodiment, a method for generating effector cells which comprises the steps of:
(i) providing effector cells or progenitor cells thereof which comprise in their genome a cassette deck structure with a cassette tape gene comprising a gene encoding the marker protein in the manner that the marker protein can be expressed in the cells and the cells do not comprise any exogenous drug resistance gene, and
(ii) exchanging the gene encoding the maker protein with a gene encoding the desired protein.

In a further embodiment, the present application also provides a method for preparing material cells having a cassette deck structure with a cassette tape gene, which comprises the steps of:
(a) preparing a vector for introducing the cassette deck structure comprising, in order from upstream to downstream, a first promoter sequence, a target sequence for a first recombinase, a gene encoding a marker protein which is linked so that it is expressed under the 1 st promoter sequence, a target sequence for the first recombinase, a target sequence for a second recombinase and a second promoter sequence that can be expressed in the material cells, and a drug resistance gene which is linked so that it is expressed under the 2nd promoter,
(b) knocking-in the vector prepared in step (a) in the material cells so that the introduced vector is expressed under the expression control system of the material cells,
(c) culturing the cells obtained in (b) in the presence of the drug to which the drug resistance gene is resistant so that the cells in which the vector for introducing the cassette deck structure with the gene encoding the marker protein has been successfully knocked-in are selected,
(d) culturing the cells selected in (c) in the presence of the second recombinase to remove the second drug resistance gene. In this embodiment, the material cells having the cassette deck structure with the cassette tape gene provided by this application can be preferably used for the cassette tape exchange by the RMCE.

In this application, "material cells" means cells before the differentiation into the effector cells. The term "material cells" encompass cells that are used for introducing a gene encoding a marker protein, and also the cells having the cassette deck structure with the cassette tape gene comprising a gene encoding the marker protein. As the material cells, pluripotent stem cells can preferably be used and more preferably, ES cells and iPS cells are used.

Examples of "effector cells" may include mature T cells and progenitor cells thereof. Examples of the marker proteins and the desired proteins may include T cell receptors (TCR) and chimeric antigen receptors (CAR). Alternatively, the marker protein may be a fluorescent protein.

In the method of the present invention, material cells having only one cassette deck structure with a cassette tape gene encoding a marker protein under the expression control system of the cell per one cell are preferably prepared. By using said material cells, a clonal population of effector cells or progenitor cells thereof having only one cassette deck structure with the cassette tape gene comprising a gene encoding the marker protein per one cell can be provided. By exchanging the cassette tape gene in the clonal population of effector cells or progenitor cells with multiple cassette tape genes, effector cells expressing different proteins can be prepared at the same time.

The present application also provides a composition for immune therapy which comprises multiple types of effector cells expressing different proteins can be prepared by the present method.

### BRIEF DESCRIPTION OF DRAWINGS

Fig. 1A is a schematic diagram of the "TIL cocktail method" provided by this application. In this diagram, RMCE is employed. When genome editing is employed, the recombinase target sequences lox2272 and loxP at the both ends of the known TCR are not required. A vector for genome editing may be applied instead of the recombinase Cre.
Fig. 1B is a schematic diagram of the "TIL cocktail method" provided by this application.
Fig. 2 is a schematic diagram of the procedures of Example 1.
Fig. 3 provides an intermediate result of example 1. The cassette deck structure KI vector was knocked-in the Jurkat cells, then the cells were treated with FLP and ganciclovir to remove the drug resistance gene.
Fig 4 provides FACS Aria charts of the cells shown in Fig. 3. TCR expressing cells were isolated.
Fig. 5A is a schematic diagram of the procedure for treating the TCR-expressing cells shown in Fig. 4, which are Jurkat cells in which the cassette tape gene comprising WT1 specific TCR gene was knocked-in, with a NY-ESO1-specific TCR containing cassette tape exchange plasmid vector and a Cre recombinase expression vector.
Fig. 5B is a schematic diagram of the same procedures as Fig. 5A except for the cassette exchange vector for knocking-in NY-ESO1-specific TCR is a linear DNA vector.
Fig. 6 provides FACS analysis of the cells obtained by treating the TCR-expressing cells shown in Fig. 4, which are Jurkat cells in which the cassette tape gene comprising WT1 specific TCR gene was knocked-in, with a NY-ESO1 containing cassette tape exchange plasmid or linear DNA vector and a Cre recombinase expression vector.
Fig. 7A provides a schematic diagram of Example 2. The step of knocking-in the cassette deck KI vector into the cells is shown.
Fig, 7B provides a schematic diagram of Example 2. The step of removing the drug resistance gene in the cassette deck KI vector from the cells.
Fig. 8 provides results of Example 2.
Fig. 9 provides results of Example 3.
Fig. 10 represents the design concept for guide RNAs used in Example 4 for cleaving the WT1 specific TCR (WT1 TCR) gene, as well as for the 5' arm and 3' arm of the desired gene KI targeting vector used for introducing the gene in the cleavage site.
Fig. 11 provides a schematic diagram of NY-ESO1 specific TCR (NYESO1-TCR) gene KI vector used in Example 4.
Fig. 12. provides a schematic diagram of CRISPR-Cas9 vector used in Example 4. Guide RNA for TCR gene cleavage (gRNA#1) and guide RNA for KI vector cleavage (gRNA #5) each operably linked to the U6 promoter, as well as a gene encoding Cas9 operably linked to the CBh promoter are included.
Fig. 13 is a schematic diagram of the knock-in process of Example 4.
Fig. 14 A provides the results of Example 4, wherein the gRNA#1 was used.
   Upper panels: Analysis for expression of TCRβ having mouse Cβ and expression of NYESO1 (NYESO1 tetramer: NYESO1). The upper numbers represent the percentage of the cells (mTCRβ+ NYESO 1 +) in the upper right fraction versus total number of the cells.
   Middle panels: Analysis of expression of TCRβ having mouse Cβ and expression of human TCR.
   Lower panels: Analysis of the expression of mouse TCRβ and expression of human TCRαβ in the mouse TCRβ-positive and NYESO1 tetramer positive cells. The number of the mouse TCRβ positive, NYESO1 tetramer positive and human TCRαβ negative cells number are shown in the bottom line.

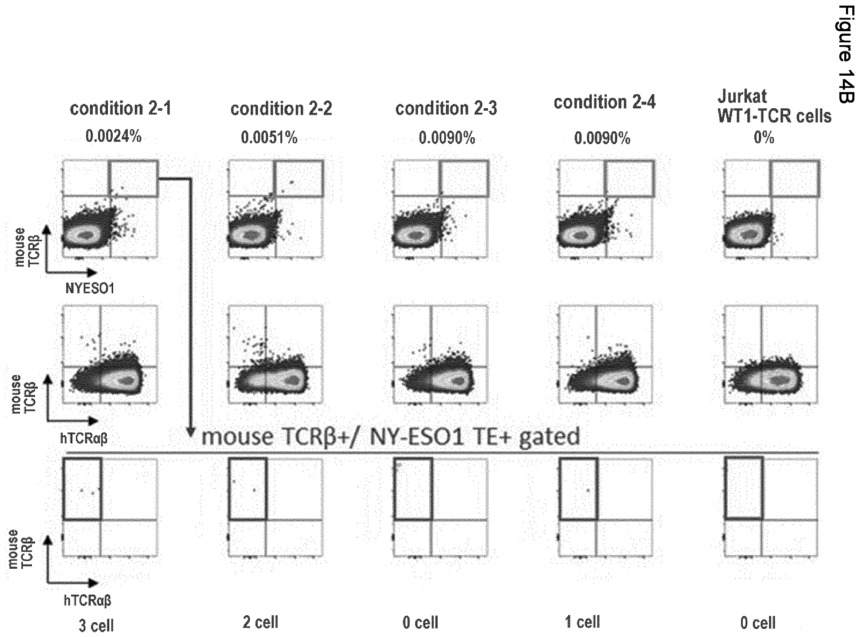

Fig. 14B provides the results of Example 4, wherein the gRNA#4 was used
   Upper panels: Analysis for expression of TCRβ having mouse Cβ and expression of NYESO1 (NYESO1 tetramer: NYESO1). The upper numbers represent the percentage of the cells (mTCRβ+ NYESO 1 +) in the upper right fraction versus total number of the cells.
   Middle panels: Analysis of expression of TCRβ having mouse Cβ and expression of human TCR.
   Lower panels: Analysis of the expression of mouse TCRβ and expression of human TCRαβ in the mouse TCRβ-positive and NYESO1 tetramer positive cells. The number of the mouse TCRβ positive, NYESO1 tetramer positive and human TCRαβ negative cells number are shown in the bottom line.

### DETAILED DESCRIPTION

In this specification and claims, "effector cell" represents a type of cell that carries out a specific activity when administered. Examples of effector cells may include various mature T cells such as helper T cells, regulatory T cells, and cytotoxic T cells, and immune cells such as Natural Killer (NK) cells, NKT cells, macrophages, and dendritic cells. In addition, platelets, hormone-producing cells, immunosuppressive cells including mesenchymal stem cells are also included in the effector cells. Further, cells administered in suspension such as nerve cells may also be effector cells. Examples of the effector cells may also include cells that form a two-dimensional tissue such as retina, retinal pigment epithelium, skin and intestinal epithelium. Furthermore, examples of the effector cells may include cells that form a three-dimensional organ such as, heart, liver, and kidney.

The method of this application may preferably be used for generating immune cells such as mature T cells, natural killer (NK) cells, NKT cells, macrophages, or dendritic cells as the effector cells. Especially, mature T cells or progenitor cells thereof having a specificity determining site, such as a specific T cell receptor (TCR) or a specific chimeric antigen receptor (CAR), as well as CAR-T cells can preferably be produced by this method.

In this application, a gene encoding a desired protein to be introduced in the effector cells are not limited to a specific TCR gene, and the gene may be any known rearranged TCR genes as well as a TCR gene obtained from T cells specific for an antigen to be targeted by an immune therapy and amplified by a known procedure. TCRs specific for a cancer antigen may be exemplified. For TIL therapy, TILs may be collected from cancer tissues of the patient, and TCRs of killer T cell clones with high frequency may be obtained by single-cell analysis of TILs and used.

The term "Chimeric Antigen Receptor" or "CAR" refers to a recombinant polypeptide construct comprising at least an extracellular antigen binding domain, a transmembrane domain, a cytoplasmic signaling domain including a cytoplasmic sequence of CD3ζ chain sufficient to stimulate T cells when bound to an antigen, and optionally one or more (for example, 2, 3 or 4) cytoplasmic costimulatory proteins that co-stimulate the T cells when the antigen binding domain is bound to the antigen. Examples of the costimulatory proteins may include CD27, CD28, 4-1BB, OX40, CD30, CD40L, CD40, PD-1, PD-L1, ICOS, LFA-1, CD2, CD7, CD160, LIGHT, BTLA, TIM3, CD244, CD80, LAG3, NKG2C, B7-H3, and a ligand that specifically binds with CD83.

The expression "progenitor cell" means a cell that is on the way of differentiation and is capable of differentiating into a specialized type of cell. The expression "material cell that is capable of differentiating into an effector cell" means cells that can be differentiated *in vitro* into the effector cells or progenitor cells thereof. The material cell is preferably a cell having abilities to differentiate into the effector cell and to proliferate *in vitro.*

Examples of the cells that are capable of differentiating into effector cells may include pluripotent stem cells and tissue-specific stem cells.

Examples of the tissue-specific stem cells or somatic stem cells may include neural stem cells, hematopoietic stem cells, mesenchymal stem cells, and dental pulp stem cells.

Pluripotent stem cells, as used herein and in the claims, are stem cells that are pluripotent, capable of differentiating into many types of cells that exist in living organisms, and that are capable of self-renewal. Pluripotent stem cells include, for example, Embryonic Stem (ES) cells, embryonic stem (ntES) cells derived from cloned embryos obtained by nuclear transfer, sperm stem cells ("GS cells"), embryonic geRMCElls ("EG cells"), Induced Pluripotent Stem (iPS) cells, and pluripotent cells derived from cultured fibroblasts and bone marrow stem cells ("Muse cells"). Pluripotent stem cells are preferably derived from mammal and preferably, from human. ES cells and iPS cells are preferably used as material cells.

The ES cells and iPS cells may be generated by a known method or the cells available on the market may be employed. iPS cells induced from the somatic cells of a patient to be treated may be used. In addition, a method for generating universal pluripotent stem cells by manipulating the HLA of ES cells or iPS cells using genome editing technology has been proposed, and the material cells of the present application may be thus obtained universal pluripotent stem cells.

As described above, the method of the present application is particularly suitable for obtaining mature T cells having a desired TCR or CAR as effector cells. As an effector cell expressing a desired protein, a method for obtaining a mature T cell expressing the desired TCR by the RMCE will be described below as an example.

First, material cells having a cassette deck structure with a cassette tape gene comprising a set of recombinase target sequences and a sequence encoding a marker protein between the target sequences in the genome of pluripotent stem cells in the manner that the marker gene is expressed when the cells are differentiated into mature T cells or progenitor cells thereof are provided.

Examples of the marker proteins may include known ligands or receptors expressed on the surface of the effector cells or progenitor cells thereof. A known TCR or CAR, a tetramer specific for which is available is preferably used as a marker protein to produce mature T cells that express the desired TCR. Alternatively, known fluorescent proteins are also exemplified as marker proteins. A large number of fluorescent proteins used for genetic engineering are known, and a variety of them are commercially available. Fluorescent proteins may be appropriately selected from these known proteins.

As used herein and in the claims, a "recombinase" is an enzyme that induces site-specific recombination, and a recombinase target sequence is a sequence that is recognized by the recombinase and is capable of inducing deletion, incorporation, or inversion of a sequence between two target sequences. Examples of combinations of recombinase and its target sequences include Cre recombinase and loxP and its derivatives, Flipperse (FLP) recombinase and frt, and clonase and attB/attP/attL/attR. The combination of the recombinase and its target sequences to be included in the cassette tape gene of the present application may be any that can be used to replace the marker gene sandwiched by the target sequences with a gene encoding the desired protein sandwiched by the same target sequences in the same direction.

For example, when Cre recombinase is employed, the target sequences may be selected from loxP, lox2272, lox511 and loxFas. Homologous recombination of those target sequences with the same target sequences with keeping the direction is promoted in the presence of Cre recombinase. Accordingly, for example a sequence in the material cell genome sandwiched by lox2272 and loxP can be exchanged with another sequence sandwiched by lox2272 and loxP in a vector.

The material cells used in the method of this application has a cassette tape gene comprising a gene encoding a marker protein in their genome in the manner that the marker protein can be expressed when the material cells are differentiated into the effector cells or progenitor cells thereof. In order to obtain mature T cells expressing a desired TCR or CAR, the cassette tape gene may be introduced into the material cells so that the gene encoding said TCR is expressed under the TCR expression system of the material cells or together with genes encoding TCR expression control system including promoter and/or enhancer.

In the description below, the structure comprising in order from upstream to downstream, a promoter, a gene encoding a marker protein, an enhancer, is referred to as a "cassette deck structure" and the gene comprising a gene encoding the marker protein is referred to as "cassette tape gene". Firstly, the procedures of step (1): preparing material cells having a cassette deck structure comprising the cassette tape gene is described below.

When the material cells have a rearranged TCR gene, for example, the material cells are iPS cells induced from a T cell, the cassette tape gene having the marker protein may be introduced under the TCR expression control system of the material cells. The cassette deck structure comprising the cassette tape gene may be constructed between the promoter and enhancer of the rearranged TCR gene locus of the material cell.

When the material cells do not have rearranged TCR gene loci, the following three types of procedures explained in the patent literature 6 (WO2020/022512) can be employed.
(A) Introduce the cassette tape gene so as to reduce the distance between the C region enhancer and the V region promoter in a TCR locus in the material cell.
(B) Introduce a sequence upstream of a C region enhancer of a TCR locus in the material cell, wherein the sequence comprises, from upstream to downstream, a V region promoter of a TCR locus and a cassette tape gene, so as to the C region enhancer and the V region promoter are sufficiently close each other to exert the TCR expression control system to express the gene sandwiched between them.
(C) Introduce a sequence downstream of a V region promoter of a TCR locus in the material cell, wherein the sequence comprises, from upstream to downstream, a cassette tape gene and a C region enhancer so as to the C region enhancer and the V region promoter are sufficiently close each other to exert the TCR expression control system to express the gene sandwiched between them.

In the context of "the V region promoter and the C region enhancer are sufficiently close each other to exert the TCR expression control system to express the gene sandwiched between them", the distance between the promoter and enhancer is not particularly limited as long as the V region promoter is controlled by the C region enhancer. It is exemplified that the distance between the V region promoter and the C region enhancer after introduction of the exogenous cassette tape gene is, for example, about 8 to 50 kbp, about 10 to 40 kbp, about 12 to 32 kbp, or about 14 to 22 kbp.

The "C region enhancer in a TCR locus" and "V region promoter in a TCR locus" each may be a sequence derived from the material cell, from cells of other individual of the same species of animal from which the material cells were derived, or from cells of other species of animal from which the material cells were derived.

The introduction of the cassette deck structure in the material cells may be conducted by an one step- or a multiple steps procedure. It can be performed by conventionally known recombination technologies, such as homologous recombination, genome editing, and a technology that uses a combination of recombinases such as Cre recombinase and Flippase recombinase.

When a rearranged TCR is used as the marker protein in the cassette tape gene, the TCR is preferably a heterodimer of TCRα and β. To express a heterodimer of rearranged TCRα and β, the gene encoding the marker protein preferably has a sequence in which the rearranged TCRα gene and TCRβ gene are connected by a self-cleaving p2A peptide. By placing the self-cleaving p2A peptide between the α and β chains, it becomes possible to express both genes under the control of a single gene expression system.

2A peptides such as p2A, T2A, E2A, and F2A can be used, and p2A peptide, which is said to have good cleavage efficiency, is suitable. Either the TCRα gene or the TCRβ gene may be introduced upstream, and the poly A sequence is suitably linked to the TCR gene which is introduced downstream.

An intron is preferably included upstream of the TCR gene. The intron may include a splice donor sequence and a splice acceptor sequence in addition to the sequence to be removed by splicing. The intron of the human polypeptide chain elongation factor alpha (EF1α) gene or the intron of the chicken beta-actin (CAG) gene promoter are exemplified.

The genes encoding TCRα and TCRβ are preferably knocked in the material cell so that the genes are under one expression control system when the material cells are differentiated into the effector cells. The TCR locus in the material cell genome at which the cassette deck structure with the cassette tape gene is knocked-in may be the TCRα or TCRβ locus. The TCRα and β locus that is not used for gene transfer may preferably be deleted. Deletion of a specific gene locus can be performed using known methods as appropriate, and can be performed using known genome editing techniques, such as CRISPR/Cas9 and Talen.

The procedures for preparing a vector for expressing a heterodimer of rearranged TCRα and TCRβ, and introducing the vector into the material cells are disclosed in Patent Literature 6 (WO2020/022512)/

The embodiment of above (B) to provide material cells for RMCE, i.e. introducing a sequence upstream of a C region enhancer of a TCR locus in the material cell, wherein the sequence comprises, from upstream to downstream, a V region promoter of a TCR locus and a cassette tape gene, so as to the C region enhancer and the V region promoter are sufficiently close each other to exert the TCR expression control system to express the gene sandwiched between them, is explained below. The material cells may be cells having a rearranged TCR gene or cells having non-rearranged TCR genes.

When the RMCE is employed, recombinase target sequences are introduced upstream and downstream flanking to the gene encoding the maker protein in the cassette tape gene. In this embodiment, a method for preparing material cells having a cassette deck structure is provided. This method comprises the steps of (a) preparing a cassette deck KI vector having, in order from upstream to downstream, a cassette tape gene comprising a first promoter that can be expressed in the effector cells when the material cells are differentiated into the effector cells, a target sequence for a first recombinase, a gene encoding the marker protein linked so as to be expressed under the first promoter, and a target sequence for the first recombinase; a target sequence for a second recombinase, a second promoter that can be expressed in the material cells, a drug resistance gene linked so as to be expressed under the second prompter, and a target sequence for a second recombinase,
(b) knocking-in the cassette deck KI vector prepared in step (a) into the material cells,
(c) selecting cells into which the cassette deck KI vector have successfully knocked in by culturing the cells in the presence of the drug to which the drug resistance gene is resistant; and
(d) causing the second recombinase to act on the cells selected in step (c) to remove the drug resistance gene flanked by the target sequence for the second recombinase.

In this embodiment, the first recombinase is the one specific for the recombinase target sequences contained in the above described material cells having the cassette deck structure. The combination of the second recombinase and its target sequence is not particularly limited. Any recombinase that does not have cross-reactivity with the first recombinase may be used. For example, when Cre/loxp system is employed for the first recombinase, the second recombinase may be FLP/frt system. The combination of the second recombinase and its target sequence may be selected so that the sequence sandwiched by the target sequences will be exchanged when the cells are cultured in the presence of the second recombinase.

The first promoter is a promoter that can induce the expression of the marker protein in the effector cells differentiated from the material cells. When the maker protein is a known TCR, the first promoter is preferably a promoter in a TCR gene locus of the material cell. A V region promoter in the TCR locus is exemplified.

In the V region of a TCR locus, there is one promoter for each V gene. The V region promoter of the TCR locus used in this embodiment is not limited and may be selected as appropriate. For example, in the case of using the TCRβ locus, the Vβ20-1 promoter is exemplified. The promoter can be obtained by amplifying the sequence by PCR with primers designed to obtain a DNA fragment of the promoter upstream from just before the translation start point in the first exon of the V gene.

The promoter that can be expressed in the material cell is not limited and may be any promoter that can induce expression of the drug resistance gene linked to it in the material cell. Examples include, but are not limited to, cytomegalovirus (CMV) promoter, simian virus 40 (SV40) promoter, and phosphoglycerate kinase (PGK) promoter. The promoter of the mouse phosphoglycerate kinase (PGK) gene (pPGK) is an example.

As a drug resistance gene, a known drug resistance gene that can function as a marker in the material cell can be used. For example, a gene resistant to hygromycin, puromycin, or neomycin may be used.

The drug resistance gene may preferably be fused with a drug-sensitive gene downstream thereof. A drug-sensitive gene is a gene that, when expressed, can induce apoptosis of the cells in response to an externally added substance. The drug-sensitive genes can be selected from known ones as appropriate, and are not particularly limited. For example, thymidine kinase genes of herpes simplex virus and varicella zoster virus may be used. Ganciclovir is an example of a substance that induces apoptosis in the cells incorporating the gene. The downstream of the drug resistance gene may preferably be linked to a poly A sequence.

As vectors used in the present application, vectors used in genetic recombination may be used as appropriate, for example, vectors such as viruses, plasmids, and artificial chromosomes. Examples of viral vectors include retrovirus vectors, lentivirus vectors, adenovirus vectors, adeno-associated virus vectors, and Sendai virus vectors. The artificial chromosome vectors include, for example, Human Artificial Chromosomes (HAC), Yeast Artificial Chromosomes (YAC), and bacterial artificial chromosomes (BAC, PAC). As plasmid vectors, plasmid vectors for mammalian cells may be used. Commercially available vectors may be selected and used according to the purpose.

In constructing the targeting vector, the site in the TCR locus in the genome of the material cell where the cassette deck structure will be introduced is determined first. The site for introduction of the cassette deck structure can be any site where the C region enhancer of the material cell can activate the V region promoter when the sequence containing in order from upstream to downstream, the V region promoter and the cassette tape gene is introduced.

For example, when a cassette deck structure is introduced into the TCRβ locus of a material cell, it is possible to introduce the gene in a region where the TCRβ locus has not been rearranged. It is possible to introduce the gene into a site close to the enhancer, such as a site upstream of Dβ2 and downstream of Cβ1. When a cassette deck structure is introduced into the TCRα locus of a material cell, it is possible to introduce the gene in a region where the TCRα locus has not been rearranged. It is possible to introduce the gene into a site close to the enhancer, such as a site upstream of the most upstream Jα gene and downstream of the most downstream Vα gene is an example.

Once the site of introduction in the TCR locus of the material cell genome is determined, sequences homologous to the sequences upstream and downstream of the introduction site are introduced as the 5' arm and 3' arm, respectively, to allow homologous recombination. In this context, a "homologous sequence" is sufficient if the sequences of the 5' arm and the 3' arm are homologous to the extent that homologous recombination occurs, respectively.

For example, in the case of introducing a cassette deck structure into a non-rearranged TCRβ locus in the genome of a material cell, a DNA fragment from about 110bp upstream of the Dβ2 gene to about 1.6kbp further upstream is used as the 5' arm sequence, and a DNA fragment from about 50bp upstream of the Dβ2 gene to about 1.6kbp downstream is used as the 3' arm sequence. DNA fragments of each 5' and 3' arm can be obtained by PCR amplification of genomic DNA of the material cell as the template using primers that can specifically amplify each sequence.

In other words, cassette deck structure knock-in targeting vector may comprise, in order from upstream to downstream, a sequence homologous to the 5' side of the introduction site in a TCR locus of the material cell genome (5' arm), a V region promoter in a TCR locus, a cassette tape gene comprising a target sequence for a first recombinase, a first promoter, a gene encoding a marker protein linked to be expressed under the first promoter, and a target sequence for the first recombinase; a target sequence for a second recombinase, a second promoter that can be expressed in the material cell, a drug resistance gene to be expressed under the second promoter, a target sequence for the second recombinase and a sequence homologous to the 3' side of the introduction site (3' arm) of the material cell is exemplified. When each sequence is amplified by PCR, the primers are designed so that the resulting PCR products include DNA sequences required for introducing the same into the drug resistance vector. The obtained PCR product can be used to construct a vector using a known method, such as the Gibson assembly method, or a commercially available kit.

The cassette deck structure KI targeting vector may further comprise in the downstream of the 3' arm a promoter that can be expressed in the material cell and a marker gene. An example of a promoter-marker combination is the combination of MC1 promoter and diphtheria toxin (DTA).

### Step (b)

The cassette deck structure KI targeting vector is knocked into a TCR locus of the material cell by homologous recombination. Knocking-in the vector can be performed by a known method, for example, by electroporation.

In order to increase the knock-in efficiency in the homologous recombination, it is preferable to introduce two single-strand breaks (nicks) near the knock-in site, e.g., near the start site (upstream) of the 3' arm, prior to knocking-in the drug resistance gene cassette knock-in targeting vector. The introduction of the nick can be performed by a known technique, and the CRISPR/Cas9n is an example.

### Step (c)

Material cells that have successfully undergone the homologous recombination express the drug resistance gene by the action of the introduced promoter. Therefore, material cells that have successfully undergone homologous recombination can be selected by culturing the cells in the presence of the drug to which the drug resistance gene is resistant. In addition, when the marker gene is eventually introduced downstream of the 3' arm of the cassette deck structure KI targeting vector, that means when the material cells are introduced with the "outer part of the 5' and 3' arm sequences" that does not contain the cassette tape gene and the drug resistance gene, the marker, for example cytotoxin, is expressed and the cells are not viable. Thus, by selecting viable cells, cells into which the cassette deck structure KI targeting vector is successfully knocked-in can be selected. The selected material cells may be further confirmed by PCR to select only the cells in which the V region promoter and the cassette tape gene are knocked in.

### Step (d)

The second recombinase is applied to the material cells into which the cassette deck structure KI targeting vector has been knocked-in. In order to apply the second recombinase, for example, the second recombinase expression vector may be introduced into the cells. By expressing the second recombinase in the cells, the drug resistance gene is removed and the material cells that have a cassette deck structure in their genome can be obtained. The cassette deck structure has a cassette tape gene comprising a promoter, a pair of recombinase targeting sequences and a gene encoding a marker protein between the recombinase targeting sequences. The material cells can be differentiated into the effector cells, and the marker protein are expressed in the effector cells or their progenitor cells when the material cells are differentiated into the effector cell or their progenitor cells.

If the introduced drug resistance gene is fused with a drug-sensitive gene, the material cells may be cultured in the presence of a factor that activates the drug sensitive gene to induce apoptosis of the cells after step (d), in order to remove cells that fail to delete the drug resistance gene.

The above explained procedure is one embodiment of the procedures conducting the step (1): providing material cells that has a cassette deck structure with a cassette tape gene containing a gene encoding a marker protein in their genome, wherein the material cells can be differentiated into the effector cells, and wherein the marker protein can be expressed in the effector cells or their progenitor cells when the material cells are differentiated into the effector cells or their progenitor cells.

For example, in order to conduct the above described embodiment (A), i.e. "introduce the cassette tape gene so as to reduce the distance between the C region enhancer and the V region promoter in a TCR locus in the material cell genome", a cassette deck structure KI targeting vector which does not comprise the first promoter sequence may be used. In order to conduct the described embodiment (C), i.e. "introduce a sequence comprising a cassette tape gene and a C region enhancer at a site downstream of a V region promoter in a TCR locus in the material cell so as to the C region enhancer and the V region promoter are sufficiently close each other to exert the TCR expression control system to express the gene sandwiched between them", a cassette deck structure KI targeting vector which does not comprise the first promoter sequence but comprises a C region enhancer of a TCR locus in the material cell in a site downstream of the drug resistance gene and a 2nd recombinase target sequence may be used.

### Step (2)

In this step, provided material cells are proliferated. Suitable procedure for proliferating the material cells may be employed based on the type of the material cells.

### Step (3)

The proliferated material cells are differentiated into the effector cells. When the material cells are pluripotent stem cells and the effector cells are T cells, the differentiation of the pluripotent stem cells into T cells may be conducted by any known procedures. For example, non-patent literatures 1 and 2 and patent literatures 1-5 disclose the differentiation procedures. By using a known procedure disclosed in a quoted prior art reference, the material cells can be differentiated into T mature cells without being destroyed the cassette deck structure construed in the material cells. Upon the differentiation, the Rag 1 and Rag 2 genes in the material cells may be deleted in order to suppress the rearrangement of the TCR. For the Rag 1 and Rag 2 genes, it is only necessary to delete one or the other.

T cells are the cells expressing CD3 and at least one of CD4 and CD8. The cells may be differentiated into either killer T cells expressing CD8 or helper T cells expressing CD4 cells based on the purpose of the treatment to be conducted with the effector cells.

The resulting T cells may comprise in their genome a cassette deck structure having, in order from upstream to downstream, a promoter, a pair of recombinase target sequences, a gene encoding a known TCR between the recombinase target sequences, and an enhancer, and express the known TCR as the marker protein.

T cells having the cassette deck structure can be selected by confirming the expression of the marker protein, the known TCR, by means of an antibody or a tetramer specific for the TCR.

By the method of disclosed in this application, a large amount of mature T cells having the same cassette tape gene comprising a gene encoding a marker protein, and expressing said marker protein can be prepared.

### Step (4):

In this step, the gene encoding the marker protein in the obtained effector cells or their progenitor cells is replaced with a gene encoding the desired protein. In one embodiment, the exchange may be conducted by means of RMCE. The obtained effector cells or their progenitor cells are cultured with a cassette tape exchange vector comprising a pair of the same recombinase targeting sequences as above and a gene encoding the desired protein in the presence of the recombinase so as to the gene encoding the marker protein is exchanged with the gene encoding the desired protein.

The cassette tape gene comprising the gene encoding the known TCR between a pair of recombinase target sequences is replaced with the cassette tape gene comprising a gene encoding a desired rearranged TCR between a pair of the same recombinase target sequences.

A gene encoding a rearranged TCR to be introduced in the T cells may be obtained by amplifying and isolating the TCR gene from T cells specific for an antigen which is a target in the cellular immunotherapy by known procedures. For TIL therapy, TILs may be collected from cancer tissues of the patient, and TCRs of highly frequent killer T cell clones may be obtained by single-cell analysis of the TILs.

Cassette tape exchange vector for TCR introduction is prepared. A TCR to be introduced in the cells is preferably a heterodimer of TCRα and β, like TCR as a marker protein. To express a heterodimer of rearranged TCRα and β, the gene encoding the TCR preferably has a sequence in which the rearranged TCRα gene and TCRβ gene are connected by a self-cleaving 2A peptide. The vector may preferably comprise, in order from upstream to downstream, intron, TCRα and β genes connected via the 2A peptide sandwiched by the recombinase target sequences, and poly A sequence.

The cassette tape exchange vector may be selected from the above explained vectors for genetic recombination. Vectors such as viruses, plasmids, and artificial chromosomes are exemplified. Linear DNA vectors can also be employed.

The cassette tape exchange vectors are introduced into the mature T cells having the cassette deck structure under the presence of the recombinase, and then, the gene encoding the TCR in the cassette tape exchange vector and the gene encoding the TCR in the material cells as a marker protein are exchanged.

The cassette tape exchange vectors and the recombinase expression vector are knocked-into the mature T cells. Knocking-in the vectors can be performed by a known method, for example, by electroporation.

By confirming the expression of the known TCR used as the marker protein in the obtained cells, cassette tape exchange can be confirmed. A known TCR can be confirmed by a tetramer or an antibody specific to the TCR.

By the method of the present application, a large amount of effector cells of the same type having one cassette deck structure with the same cassette tape gene per cell can be prepared. A desired protein can be expressed in the effector cells by exchanging the cassette tape gene.

The method of the present application can be conducted by using genome editing. When genome editing is employed, the material cells having the cassette deck structure provided in step (1) may be the cells that have a cassette deck structure with a cassette tape gene comprising a gene encoding a marker protein in their genome, wherein the material cells can be differentiated into the effector cells, and wherein the marker protein can be expressed in the effector cells or their progenitor cells when the material cells are differentiated into the effector cells or their progenitor cells. In this context, the "cassette deck structure" comprises, in order from upstream to downstream, promoter, a gene encoding the marker protein, and enhancer, and the "gene encoding the marker protein" corresponds to the "cassette tape". When the genome editing is employed, it is not necessary to sandwich the gene encoding the marker protein by recombinase target sequences.

Steps (2) and (3) are the same as those when the RMCE is employed.

Genes encoding the desired protein are introduced in the effector cells obtained in step (3) by means of genome editing so as to destroy the gene encoding the marker protein and introduce the gene encoding the desired protein, or so as to exchange the gene encoding the marker protein with the genes encoding the desired proteins so that the genes encoding the desired proteins can be expressed in the material cells. In one embodiment, CRISPR/Cas9 system, one of the genome editing techniques, may be used to destroy the gene encoding the marker protein and introduce another rearranged TCR.

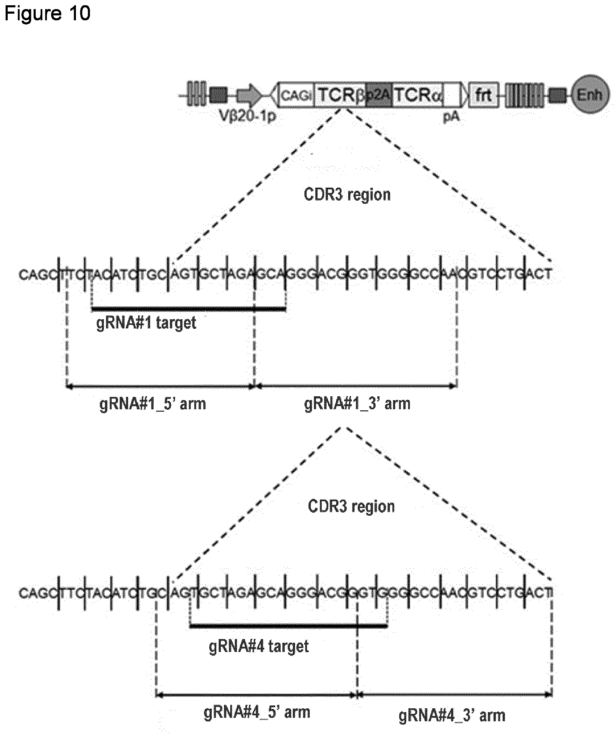

### i) Design of guide RNA for marker protein gene cleavage (see Fig. 10)

The guide RNA is designed so that a site in the genome that affects the specificity of the marker protein is cleaved. For example, when the marker protein is a TCR and the gene encoding the same comprises TCRα and TCRβ genes connected by 2A, the guide RNA may be designed so that one of CDR1, CDR2 and CDR3 of either one of the TCR chains, which are the regions affecting the specificity of the TCR, is cleaved. One embodiment in which CDR3 in the TCRβ chain variable region is cleaved is explained below.

### ii) Design of the arms for rearranged TCR gene KI vector (see Fig. 10).

The guide RNA recognition site or the CRISPR/Cas9 cleavage site in the CDR3 region is determined, and the sequences from the cleavage site to 20bp upstream and to 20bp downstream are identified as 5' and 3' arm homologous sequences respectively.

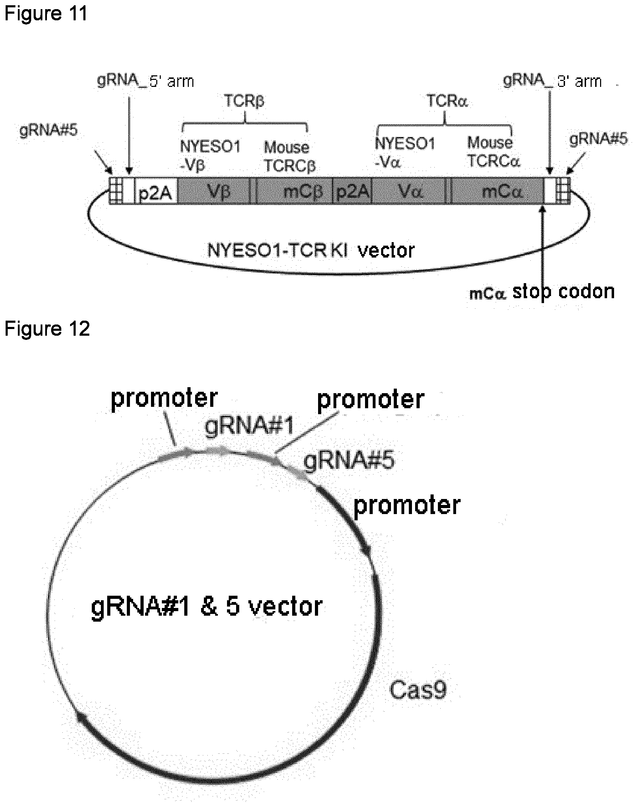

### iii) Design of the rearranged TCR gene KI vector (see Fig. 11)

Then, a KI vector for introducing the TCR is designed. When a heterodimer of rearranged TCRα and β is knocked in, the gene encoding the TCR may preferably be a gene encoding an amino acid sequence in which the rearranged TCRα and TCRβ are connected by the self-cleaving 2A peptide. The sequence to be knocked-in is sandwiched by the 5' and 3' arm homologous sequences identified in step ii) and KI vector cleavage guide RNA recognition sites are placed on the both ends.

A gene encoding the self-cleavage 2A peptide is placed upstream of the knock-in sequence flanked by the 5' and 3' arms, and a termination codon is placed downstream of the gene encoding the desired protein. In one example, the rearranged TCR gene KI vector comprises a KI vector cleavage guide RNA recognition sequence, a 5' arm sequence, a gene encoding 2A peptide, a gene encoding TCRβ, a gene encoding 2A peptide, a gene encoding TCRα, a termination codon, a 3'arm sequence, and a KI vector cleavage guide RNA recognition sequence.

### iv) Preparation of a CRISPR/Cas9 vector

Expression vector which can express Cas9 together with a guide RNA for cleaving the gene encoding the marker protein and a guide RNA for cleaving the rearranged TCR gene KI vector, separately or together is prepared. The vector can be prepared by inserting the two guide RNA sequences into a commercially available CRISPR/Cas9 vector downstream of each promoter so that the guide RNAs are operably linked to the promoter. Fig. 12 shows a schematic diagram of a vector into which the two guide RNAs have been inserted. Examples of commercially available Cas9 gene expression vectors include pX330, pCAS-Guide, and pGuide-it.

### v) Knocking in the rearranged TCR gene KI vector and the CRISPR/Cas9 vector into the material cells

The vectors may be knocked-in the material cells by a known method, for example, electroporation.

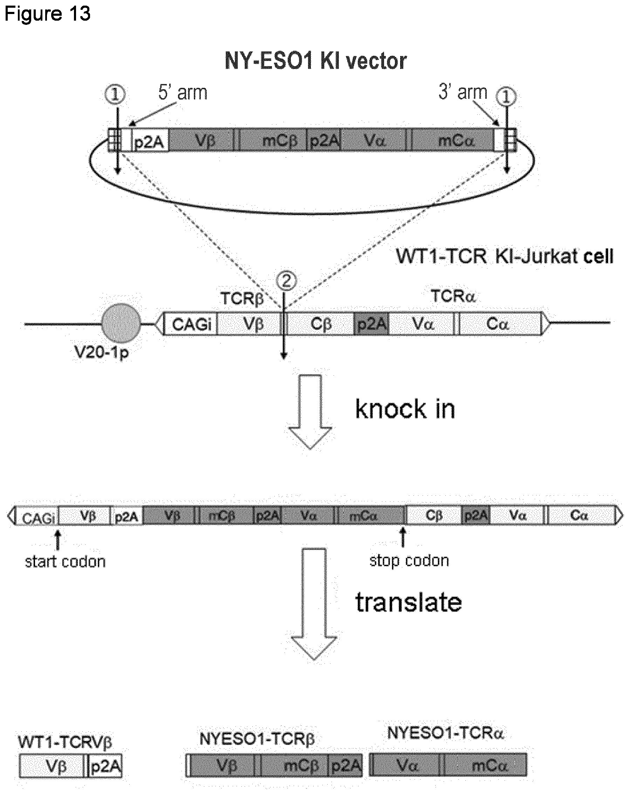

By knocking-in the vectors, the gene encoding the rearranged TCR is inserted in the manner interrupting the specificity controlling region in the TCR gene, the gene encoding the marker protein (Fig. 13). In Fig. 13, the sequence from the start codon in the gene encoding the marker protein in the material cell to the stop codon in the KI rearranged TCR gene is translated. The 5'-side fragment of the marker TCRβ chain is cleaved off by self-cleavage of the p2A peptide and loses its specificity. The rest of the marker TCR is 3' side from the stop codon at the end of the KI rearranged TCR gene and is not translated. The translated rearranged TCR α and β chains are generated as two proteins by self-cleavage of the intervening p2A peptide. Thus, the TCR used as the marker protein is replaced with the desired rearranged TCR.

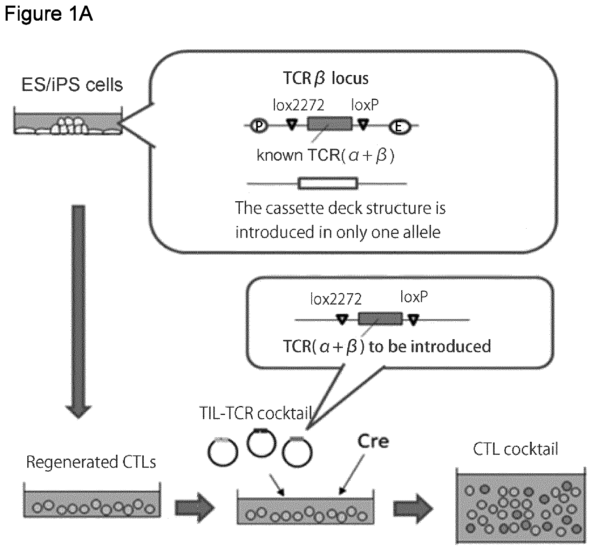

By the method of the present application, a large amount of mature T cells or progenitor cells having the cassette deck structure with a cassette tape gene comprising the gene encoding a known rearranged TCR can be prepared, and then the cassette tape gene in the material cells can be replaced with a cassette tape gene having a gene encoding a desired TCR. By providing mature T cells or their progenitor cells each having one cassette deck structure per one cell, the cassette tape gene in a plurality of mature T cells or their progenitor cells can be exchanged with multiple TCR cassette tape genes simultaneously (Fig. 1A). For example, by destroying the TCR expression system that does not have the marker protein, it will be possible to provide the effector cells having only one cassette deck structure per one cell. The method wherein the cassette tape gene in clonal population of effector cells having only one cassette deck structure is exchanged with multiple TCR cassette tape genes will be preferably employed in the TIL therapy.

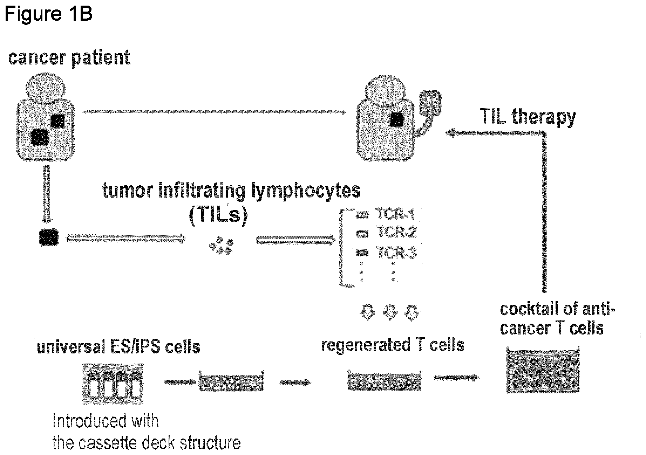

When the method of this application is used for the TIL therapy, a cocktail method in which cassette tape gene in clonal cells having only one cassette deck structure in one cell is exchanged with multiple TCR cassette tape genes is exemplified. First, TILs are obtained from the patient, multiple high frequency killer T cell clones in the TILs are identified by single cell analysis, and then information of the TCRs in the Killer cell clones are determined. Genes encoding thus obtained multiple TCRs are introduced in the cassette tape exchange vectors (Fig. 1B). On the other hand, universal pluripotent stem cells obtained by manipulating their HLAs may be used as material cells for producing the effector cells. Clonal population of a T cell having the cassette deck structure may be regenerated from the material cell and, and the desired TCRs are introduced into the regenerated T cells. In the step of introducing TCRs in the T cells having the cassette deck structure, the method of this application may be employed. This strategy is theoretically applicable to all patients from which TILs can be obtained. This embodiment is a combination of universal and individualized therapy, i.e., the T cells are for universal use and TCRs are individually derived.

Techniques for manipulating HLA of ES cells or iPS cells to provide universal pluripotent stem cells have been proposed and in one embodiment, the material cells used in the method provided in this application may be the universal pluripotent stem cells.

When the RMCE is employed, the TIL cocktail method may have following features:
1) A gene encoding a rearranged TCR is introduced at a site in a TCR gene locus of ES/iPS cells so that the expression of the introduced TCR gene is under the control of the endogenous enhancer (Fig. 1A).
2) Lox2272 and loxP are placed in the genome so that the upstream and downstream of the introduced TCR gene is respectively flanked by those sequences.
3) A known rearranged TCR to which a specific tetramer is available can be used in this embodiment. The tetramer can be used for the tetramer staining.
4) The TCR gene locus in the cells other than the TCR locus into which the rearranged TCR gene has been introduced is destroyed so that only one TCR locus in the cells is expressed.
5) Thus prepared ES/iPS cells are differentiated into T cells.
6) On the other hand, lox2272 and loxP are placed upstream and downstream of the desired TCR gene to be introduced in the cells to provide cassette tape exchange vectors,
7) Multiple cassette tape exchange vectors are introduced into the T cells together with Cre recombinase.

When genome editing is employed, the TIL cocktail method may have the following features:
1) A gene encoding a rearranged TCR is introduced at a site in a TCR gene locus of ES/iPS cells so that the expression of the introduced TCR gene is under the control of the endogenous enhancer.
2) The TCR gene locus in the cell other than the TCR locus into which the rearranged TCR gene is introduced is destroyed so that only one TCR locus in the cells is expressed.
3) A known rearranged TCR to which a specific tetramer is available can be used in this embodiment. The tetramer can be used for the tetramer staining.
4) Thus prepared ES/iPS cells are differentiated into T cells.
5) On the other hand, a vector for genome editing used for introducing the gene encoding the rearranged TCRs at a specific site and cassette tape exchange vectors which are used for introducing the desired rearranged TCR genes are prepared.
6) Multiple cassette tape exchange vectors are introduced into the T cells together with the vector for genome editing.

By those embodiments, there are incredible effects, including 1) a mixture of cassette tape exchange vectors comprising different TCR genes can be used for gene transfer without risk of mispairing, and 2) The T cells whose TCR have been successfully exchanged can be isolated as a tetramer negative cell population. As a result, large amounts of T cells expressing multiple TCRs that are similar to those in the TILs can be generated.

On the other hand, the present invention can also be employed for introducing one or multiple genes encoding TCR or CAR specific to a cancer antigen in the regenerated T cells.

### EXAMPLE 1

The present application will be described in more detail referring to the examples below. In the examples, a cassette tape gene having a specific gene is referred to as "specific gene cassette tape".

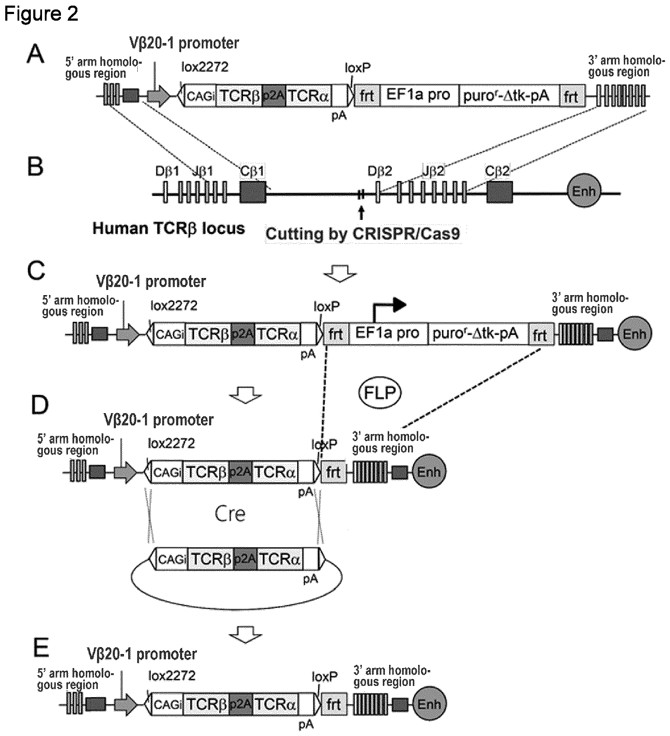

In the examples of the present application, Jurkat cells having a cassette deck structure with a WT1 specific TCR gene cassette tape under the endogenous TCR expression control system were prepared. The WT1 specific TCR gene cassette tape in the cassette deck structure in the cells was exchanged with a NY-ESO1 specific TCR gene cassette tape by treating the cells with the NY-ESO1 specific TCR gene cassette tape exchange vector. The scheme of this example is shown in Fig. 2. 1) Knocking-in of a cassette deck structure with a TCR gene cassette tape into the Dβ2 region on the TCRβ locus in the Jurkat cells by homologous recombination.

### REAGENTS and ANTIBODIES:

KOD-Plus-Neo (Toyobo, KOD-401),
Amaxa^{®} Cell Line Nucleofector^{®} Kit V (Lonza, VACA-1003),
Puromycin dihydrochloride (Wako,160-23151)
ganciclovir (Wako,078-04481)
PE/Cy7 anti-human TCRα/βmonoclonal antibody (BioLegend, 306719),
APC Anti-Human CD3 monoclonal antibody (BioLegend, 300439),
APC/Cyanine7 anti-human CD3 Antibody (BioLegend, 300426)
APC mouse IgG2bκ isotype control antibody (BioLegend, 400322),
HLA-A*24:02 modified WT1 Tetramer-CYTWNQMNL-PE (MBL, TS-M014-1),
HLA-A*02:01 NY-ESO-1 Tetramer-SLLMWITQC-PE (MBL, TB-M011-1)

The culture medium shown below was used for cell culture in all examples. In the case of selection by an agent, each agent was added to the medium and cultured.

**[Table 1]**

| Cell culture medium | (final concentration) |
|---|---|
| RPMI1640 | 500 mL |
| FCS | 50 mL(9%) |
| * penicillin/streptomycin/L-glutamine solution | 5.55 mL(1%) |
| 2-mercaptoethanol | 2 µL(50 µM) |
| Total | 555 mL |

| | |
|---|---|
| *The composition of the penicillin/streptomycin/L-glutamine solution is 10,000 U/mL of penicillin, 10,000 µg/mL of streptomycin, and 29.2 mg/mL of L-glutamine, so the final concentrations are 100 U/mL, 100 µg/mL, and 292 µg/mL, respectively. | |

The vector was introduced into the cells by electroporation. Electroporation was performed according to the manual of the Amaxa^{®} Cell Line Nucleofector^{®} Kit V. The cells and the vector were suspended in the reagents provided in the kit, the suspension was transferred to the cuvette provided in the kit, the cuvette was set in the Amaxa Nucleofector II (Lonza), and the vector was introduced into the cells using the built-in program X001.

J.RT3-T3.5 Jurkat cells, a variant of the Jurkat cell line with impaired expression of the endogenous TCRβ gene (hereafter referred to as Jurkat β mutant) (Ohashi et al., Science 316, 606-609, 1985) were used as the material cells.

A targeting vector shown in Fig. 2A was used as the cassette deck structure knock-in targeting vector. The vector comprised a cassette tape gene having a rearranged TCR gene, which corresponds to the marker protein, flanked by lox2272 and loxP. The vector also had a DCT gene and a promoter for the expression of the gene downstream of the 3' arm homologous region (not shown in Fig. 2A).

The Vβ20-1 promoter and the TCR gene cassette tape were introduced into the approximately 50 bp upstream of the Dβ2 gene on the TCRβ locus (Fig.2B) of the Jurkat cells, a T cell line, using the cassette deck structure KI targeting vector (KI targeting vector) (Fig. 2A). A schematic diagram of the material cell after KI is shown in Fig. 2C.

To increase the knock-in efficiency by homologous recombination, two single-strand breaks (nicks) were introduced at a site approximately 50bp upstream of the Dβ2 gene by the CRISPR/Cas9n system. KI targeting vector was introduced into the Jurkat β mutant along with the two CRISPR/Cas9n vectors shown below.

In the cells that have been incorporated with the cassette deck structure KI targeting vector into the their genomic DNA by homologous recombination, puromycin resistance gene fused with the kinase domain of herpesvirus thymidine kinase (ΔTK) (PurorΔ TK) will be expressed by the action of EF1α promoter (Fig. 2C). Accordingly, clones that incorporated the drug resistance gene in the cassette deck structure KI targeting vector into their genome were selected using 0.25µg/mL puromycine/culture medium (positive selection). On the other hand, the cells into which the outer portion of the 5' arm and 3' arm of the drug resistance gene KI targeting vector was incorporated by random integration could not survive because diphtheria toxin (DTA) is produced intracellularly (negative selection), and were removed.

Then, from the clonal cell population so selected, cells that were incorporated with the part from the Vβ20-1 promoter to frt including the TCR gene and the drug resistance gene on the TCRDβ2 locus were identified by PCR. Thus, the clone into which the TCR gene cassette tape vector was introduced in only one allele of the Dβ2 region and the vector was not incorporated into the other region in their genomic DNA was selected by PCR.

### Materials

**[Table 2]**

| | | |
|---|---|---|
| cells | Jurkat β mutant | 2×10⁶ cells |
| vectors | cassette deck structure KI targeting vector | 1.0 µg |
| | CRISPR/Cas9n vector 1 | 0.5µg |
| | CRISPR/Cas9n vector 2 | 0.5 µg |
| agent | Amaxa^{®} Cell Line Nucleofector^{®} Kit V | 100 µL |

CRISPR/Cas9n vectors were prepared by using the following oligonucleotides:
A: 5'-CACCGAGGTTAGTCTGACTGTGTG-3' (SEQ ID NO: 1)
B: 5'-AAACCACACAGTCAGACTAACCTC-3' (SEQ ID NO: 2)
C: 5'-CACCCTGCCGCTGCCCAGTGGTTG-3' (SEQ ID NO: 3)
D: 5'-AAACCAACCACTGGGCAGCGGCAG-3' (SEQ ID NO: 4)

Oligonucleotides A and B (vector 1), and oligonucleotides C and D (vector 2) were annealed, respectively, and then, introduced into plasmid pX460 cleaved with the restriction enzyme Bbs1.

The 5' arm and 3' arm regions were amplified by PCR with the primers as shown below to confirm that the TCR gene was incorporated on the TCRDβ2 locus of the cells.

Primers used in the example are as follows:
For 5' arm region
   Primer 1, 5'-ACGGCTGAAATCTCCCTAACCC-3' (SEQ ID NO: 5)
   Primer 2, 5'-ATACGAAGTTATAGCTAGTCTTCCGTGATGGCCTCACACCA-3' (SEQ ID NO: 6)
   PCR was performed by using KOD-FX (Toyobo, KFX-101) at 94°C, 2 min. → [98°C, 10 sec. → 68°C, 4 min.] for 35 cycles.
For 3' arm region
   Primer 3, 5'-GTCCAGACCCACGTCACC-3' (SEQ ID NO: 7)
   Primer 4, 5'-GGGGACCGAGGGGCTGGAAG-3' (SEQ ID NO: 8)
   PCR was performed by using KOD-FX (Toyobo, KFX-101) at 94°C, 2 min. → [98°C, 10 sec. → 68°C, 2 min. 30sec.] for 35 cycles.

In order to confirm that the TCR gene was incorporated in only one allele of the TCRDβ2 region, PCR was conducted with the following primers:
Primer 5, 5'-CCTCCTGTCATAAGGTGCCAT-3'(SEQ ID NO: 9)
Primer 6, 5'- CCACTTTGCTGTCTTGGCCTT-3' (SEQ ID NO: 10)
PCR was performed by using KOD-FX (Toyobo, KFX-101) at 94°C, 2 min. → [98°C, 10 sec. --+ 60°C, 30sec. → 68°C, 8 min.] for 35 cycles.

In order to confirm whether the vector was incorporated into the genomic DNA by random integration, diphtheria toxin (DTA) gene was amplified by PCR with the following primers:
Diphtheria toxin (DTA) gene
Primer 7, 5'-AGCCAAAATCTGGTACACAAGG-3' (SEQ ID NO: 11)
Primer 8, 5'-CTGAGCACTACACGCGAAGCA-3' (SEQ ID NO: 12)
PCR was performed by using KOD-FX (Toyobo, KFX-101) at 94°C, 2 min. → [98°C, 10 sec. → 58°C, 30 sec. → 68°C, 25 sec.] for 35 cycles.

The cells containing no diphtheria toxin were used as "cassette deck structure KI targeting vector KI-Jurkat cells" in the following procedures.

### 2) Introduction of FLP vector into cassette deck structure KI targeting vector KI-Jurkat cells (creation of exogenous TCR-expressing Jurkat cells)

### Materials

**[Table 3]**

| | | |
|---|---|---|
| Cell | cassette deck structure KI targeting vector KI-Jurkat cells | 2×10⁶ cells |
| Vector | FLP expression vector (pCAGGS-FLPe) | 5.0 µg |
| Reagent | Amaxa^{®} Cell Line Nucleofector^{®} Kit V | 100 µL |

The Flipparse expression vector (pCAGGS-FLPe) was introduced into the cassette deck structure KI targeting vector KI-Jurkat cells by electroporation. FLP recognizes frt sequences and delete the region flanked by them. Fig. 2C and 2D show the Dβ2 region on the TCRβ gene locus after the knock-in (Fig. 2C) and the TCRβ gene locus with deletion of the portion flanked by frt sequences (Fig. 2D).

The transient FLP expression by electroporation, cells in which the region flanked by frt was deleted coexist with cells without the deletion. Therefore, ganciclovir was used to eliminate cells that failed to delete the region flanked by frt, i.e. cells failed FLP recombination. Ganciclovir usually has no effect on human cells, such as Jurkat cells. In contrast, in cells where PurorΔTK is present, ganciclovir becomes a nucleic acid analog when phosphorylated by ΔTK, resulting in inhibition of DNA replication and finally, in cell growth arrest or cell death.

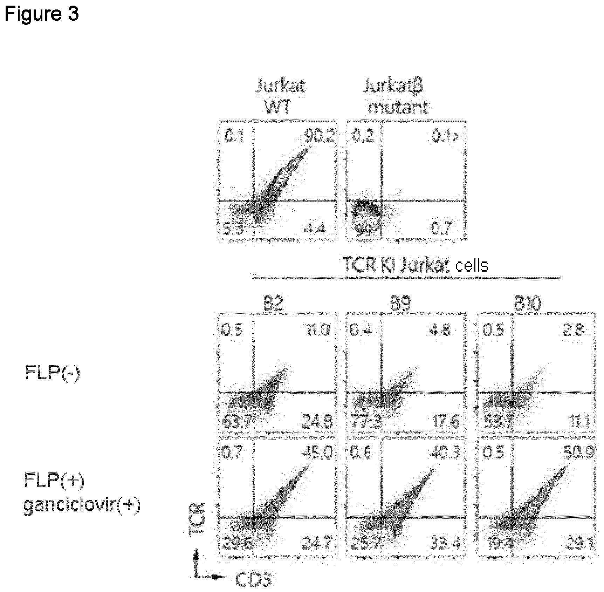

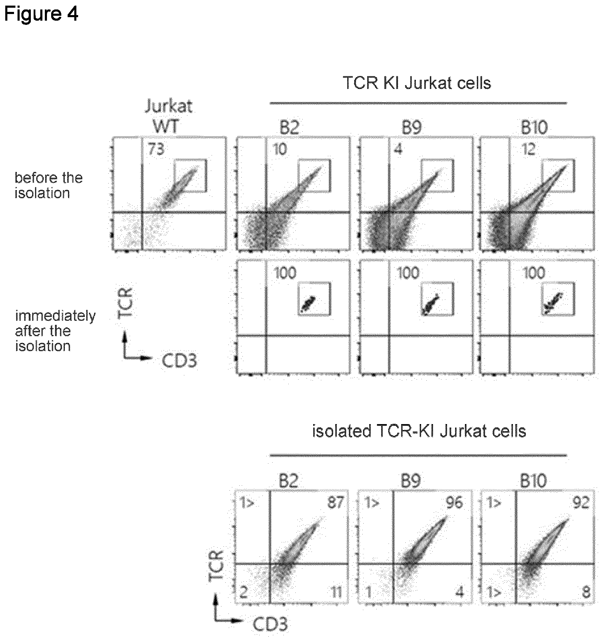

The cells were cultured with 12 µM ganciclovir/medium for 9 days and then, the expression of TCR and CD3 on the cell membrane was analyzed by FACS. The results are shown in Fig. 3. Ganciclovir selection increased TCR-expressing cells. However, many cells expressing no TCR were included. Then cells expressing TCR were isolated using FACAAria (BD) and used below (Fig. 4).

The isolated cells were cultured, the deletion of the region flanked by frt sequences was confirmed by PCR, and reanalyzed by FACS. It was confirmed that the isolated cells lacked the region flanked by frt sequences and maintained the TCR expression (Fig. 4). The obtained cells were used in the following experiments as "TCR expression cassette deck KI-Jurkat cells".

### 3) TCR cassette tape exchange in TCR expression cassette deck KI-Jurkat cells using a plasmid DNA

### Materials

**[Table 4]**

| | | |
|---|---|---|
| Cell | TCR cassette deck KI-Jurkat cells | 2×10⁶ cells |
| Vectors | TCR cassette tape exchange vector (NY-ESO1) | 1.0 µg |
| | Cre recombinase expression vector (pCAG-nls-Cre) | 4.0 µg |
| Reagent | Amaxa^{®} Cell Line Nucleofector^{®} Kit V | 100 µL |

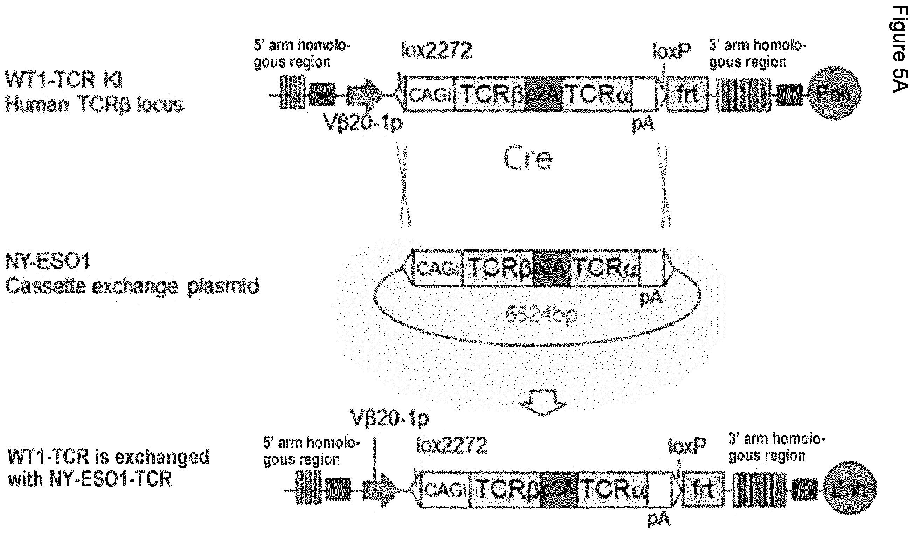

Procedures are shown in Figs. 2D and 2E. The procedures are again summarized in Fig. 5A. The upper panel shows the Dβ2 region in the TCRβ locus of the TCR cassette deck KI-Jurkat cell. The middle panel shows TCR cassette tape exchange plasmid vector. The lower panel shows a schematic view of the TCRβ locus after the cassette tape exchange. There is a sequence containing intron-TCRβ-p2A-TCRα-poly A flanked by Lox2272 and Loxp in the downstream of V020-1 promoter in the Dβ2 region on the TCRβ locus.

A plasmid vector containing the sequence of intron-TCRβ-p2A-TCRα-poly A flanked by Lox2272 and Loxp was used as the TCR cassette tape exchange vector. TCRα and TCRβ specific for NY- ESO 1 antigen were used.

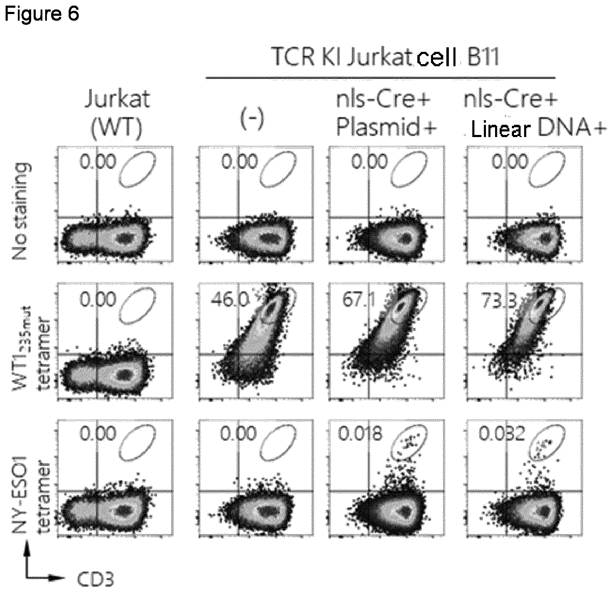

In order to exchange the cassette tape, the TCR cassette tape exchange vector and Cre recombinase expression vector (pCAG-nls-Cre) were introduced in the TCR cassette deck I-Jurkat cells by electroporation. The cells were cultured for seven days after the electroporation. In order to confirm that the region flanked by lox2272 and loxP was exchanged by this cassette tape exchange procedure, the presence of NY-ESO1 tetramer positive cells were confirmed by FACS. Results are shown in Fig. 6. In the cells that were not subjected to the cassette tape exchange procedure, no NY-ESO1 tetramer positive cells was confirmed. In the cells introduced with the TCR cassette tape exchange vector and Cre expression vector, the presence of NY-ESO1 tetramer positive cells were confirmed.

### 4) TCR cassette tape exchange procedure in the TCR expressing cassette deck KI-Jurkat cells by using a linear DNA vector.

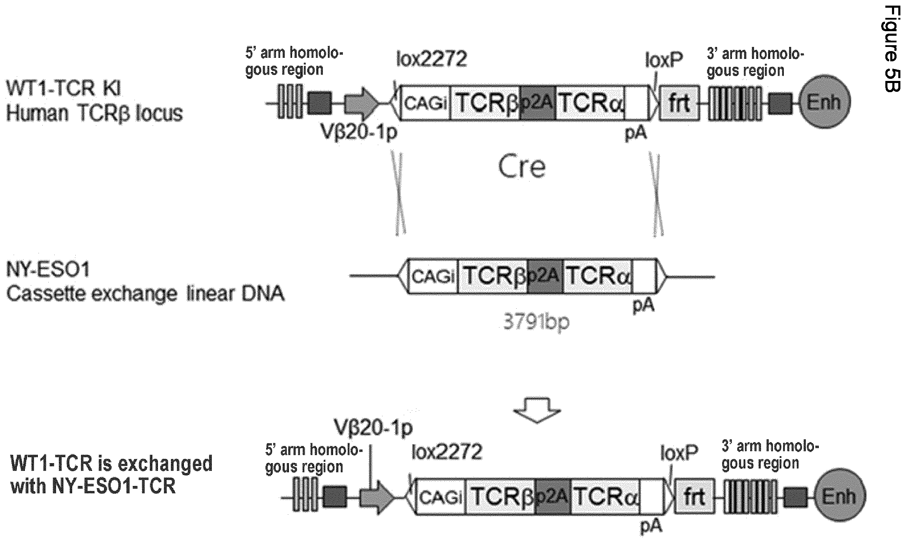

In order to increase the efficiency of the cassette tape exchanging procedures, a linear DNA vector whose size is smaller than the plasmid DNA vector was used in the cassette exchange procedure. The schematic diagram of the procedure is presented in Fig. 5B. The linear DNA vector was prepared by amplifying the region flanked by lox2272 and loxP of the above described TCR cassette tape exchange vector by means of PCR. See materials shown below. The linear DNA vector for TCR cassette tape exchange and Cre recombinase expression vector (pCAG-nls-Cre) were introduced in the TCR cassette deck KI-Jurkat cells by electroporation. The cells were cultured for fourteen days after the electroporation. In this example, NY-ESO1 specific TCR was knocked in. In order to confirm that the region flanked by lox2272 and loxP was exchanged by this cassette tape exchange procedure, the presence of NY-ESO1 tetramer positive cells were confirmed by FACS. Results are shown in Fig. 6. NY-ESO1 tetramer positive cells was confirmed in the cells introduced with the linear DNA TCR cassette tape exchange vector and Cre expression vector, and the presence of NY-ESO1 tetramer positive cells were confirmed. The results were similar to those obtained when the plasmid vector was used.

### Materials

Linear DNAs for TCR cassette tape exchange
Primer 9, 5'-TGTAAAACGACGGCCAGT-3' (SEQ ID NO: 13)
Primer 10, 5'-CAGGAAACAGCTATGACCATG-3' (SEQ ID NO: 14)
PCR was performed by using KOD-FX (Toyobo, KFX-101) at 94°C, 2 min. → [98°C, 10 sec. → 59.1°C, 30 sec. → 68°C, 4 min.] for 35 cycles.

### Materials

**[Table 5]**

| | | |
|---|---|---|
| Cell | TCR cassette deck KI-Jurkat cells | 2×10⁶ cells |
| Vectors | Linear DNA for TCR cassette tape exchange (NY-ESO1) | 1.0 µg |
| | Cre recombinase expression vector (pCAG-nls-Cre) | 4.0 µg |
| Reagent | Amaxa^{®} Cell Line Nucleofector^{®} Kit V | 100 µL |

### EXAMPLE 2

### 1) Knocking-in of a cassette deck structure with a WT-1 specific TCR gene flanked by lox sites (cassette tape gene) into the Dβ2 region of the TCRβ locus in human iPS cells

### Materials

**[Table 6]**

| | | |
|---|---|---|
| Cell | Human iPS cell clones HKM1, 114s04 | 1 × 10⁶ cells |
| Vectors | 1. pX330-hTCRDβ2-gRNA #1 (Crispr/CAS9n and guide RNA expression vector) | 3.3 µg |
| | 2. KM3-3Db2K TCR cassette deck structure KI targeting vector (Fig. 7A) | 4.0 µg |
| | 3. pCAGGS-FLP | 10 µg |
| Reagents | Opti-MEM (Thermo Fisher) | |
| | iMatrix-511 (nippi) | |
| | Puromycin (Gibco) | |
| | Ganciclovir (Wako) | |
| Medium | AK03N (Ajinomoto) | |
| Devise | NEPA21 (NEPAGENE) | |

The following iPS clones were used
HKM1: Human iPS cell line established from human monocyte at Kawamoto laboratory of Institute for Frontier Medical Sciences, Kyoto University
I14s04: Human iPS cell line established from human monocyte at Center for iPS Cell Research and Application, Kyoto University.

### 2) Knocking-in of the TCR by genome editing with CRISPR/Cas9

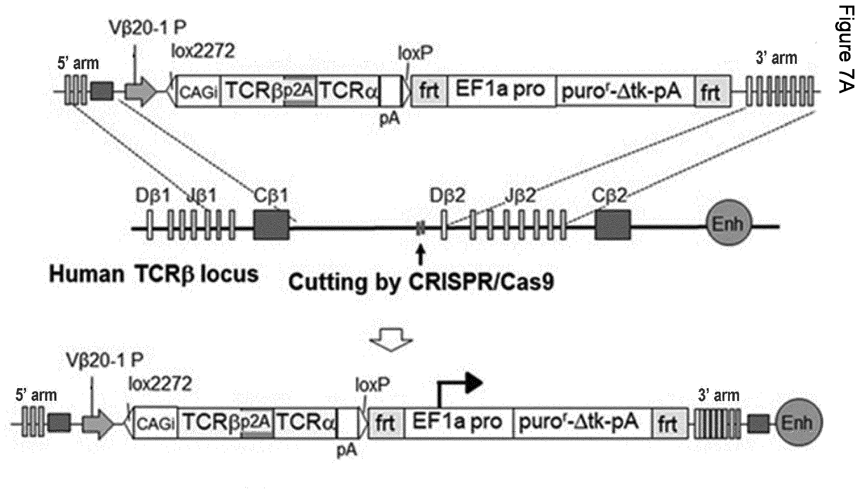

A cassette deck structure KI targeting vector was prepared in the same manner as Example 1. The structure of the vector is shown in Fig. 7A. The TCR gene used here was KM#3-3 prepared from WT1 specific TCRs. The plasmid obtained by mixing the 1: CRISPR/CAS9n and guide RNA expression vector and 2: TCR cassette deck structure KI targeting vector shown in table 6 were transfected in the iPS cells by electroporation. Two days after the electroporation, the cells were collected and re-seeded on a 6-well plate at 5×10⁴cells/well. One day after, the plate was then subjected to the puromycin selection at the final concentration shown below.

Electroporation was conducted with 1 × 10⁶ iPS cells and plasmid DNA in total 10 µg per one time. The procedures was conducted by using NEPA21 according to the criteria shown below:
Day 0: Electroporation
Day 1: medium exchange
Day 2: re-seeding the cells at 5×10⁴ cells/well
Day 3: puromycin 180 ng/ml
Day 4: puromycin 150 ng/ml
Day 5-9: normal culture
Day10: pick up iPSC colonies

Genomic DNAs of thus transfected iPS cells were analyzed by PCR and clones in which the desired knock-in was achieved were confirmed and selected.

### 3) Deletion of the structure for drug selection

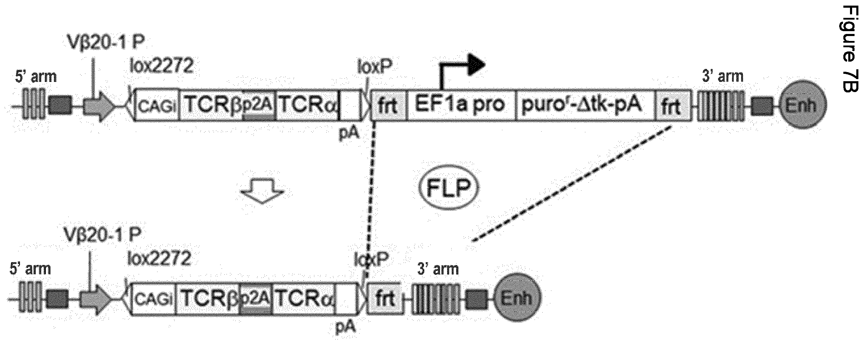

Thymidine kinase was expressed downstream of the cassette deck structure KI targeting vector shown in Fig. 7A, and ganciclovir (GCV) was made to be toxic in the cells due to the enzyme. Therefore, the cells die when co-cultured with GCV. Using this mechanism, 3: FLP expression vector is transiently introduced by electroporation and after the transfection, GCV at the final concentration shown below was added to select the cells. The schematic diagram is shown in Fig. 7B.
Day 0: electroporation
Day 2: reseeding the cells at 2-4×10³ cells /well
Day 3-6: GCV 5µg/ml
Day 7-12: Normal culture
Day 12: pick up the iPSC colonies

Genomic DNA of thus transfected iPS cell clones were analyzed by PCR and confirmed whether the PurorΔTK region was deleted.

CD8 single positive T cells or cytotoxic Tcells (CTL) were differentiated from the iPS cells having the cassette deck structure with the lox2272-TCR-loxP motif wherein the TCR is the WT1 specific TCR.

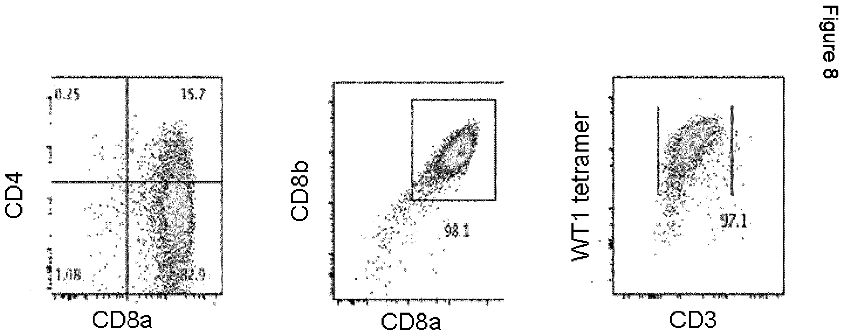

Differentiation of the iPS cells into CTLs were conducted by the method described in Patent Literature 4 (WO2017/179720). Namely, iPS cells were differentiated into T cell progenitors which are CD4CD8 double positive cells, then the CD4CD8 double positive cells were isolated. The isolated CD4CD8 double positive cells were further differentiated into CD8 single positive cells. Results are shown in Fig. 8. WT1 specific CD8 single positive cells having a heterozygous type CD8 antigen comprising CD8 α and CD8 β chains were obtained.

### EXAMPLE 3

CD8 single positive T cells having the cassette deck structure with the WT1 specific TCR cassette tape obtained in Example 2 were subjected to the cassette tape exchange 1) TCR cassette tape exchange in CD8SP T cells by using plasmid DNA

**[Table 7]**

| | | condition | |
|---|---|---|---|
| | | (1) | (2) |
| Cells | regenerated CTLs | 1×10⁶ cells | |
| | (CD8SP T cells having a cassette deck structure with the WT1 specific TCR cassette tape) | | |
| Plasmid | TCR cassette tape exchange vector (NY-ESO1) | 0.8µg | 0.5µg |
| | Cre expression vector(pCAG-nls-Cre) | 0.2µg | 0.5µg |
| reagents | αMEM medium supplemented with 20% FBS(Gibco) | | |
| | anti-CD3 antibody (Invitrogen) | 1µg /ml | |
| | anti-CD28 antibody (Invitrogen) | 1µg /ml | |
| | RetroNectin^{®} (TAKARA Bio) | 5µg /ml | |
| | hIL-2 (Peprotech) | 10ng/µl | |
| | hIL-7 (Peprotech) | 5ng/µl | |
| | hIL-15 (Peprotech) | 10ng/µl | |
| | hIL-21 (Peprotech) | 10ng/µl | |
| | P3 Primary Cell 96 well Nucleofector^{®} kit (Lonza) | | |
| devise | 4D-Nucleofector (Lonza) | | |

Cassette tape genes were exchanged according to the procedures shown in Fig. 5A. The cassette tape exchange vector was a circular plasmid vector comprising a sequence of intron-TCRβ-p2A-TCRα-poly A flanked by lox2272 and LoxP. TCRα and TCRβ were specific for the antigen NY-ESO1. A vector that can express GFP gene flanked by lox2272 and Lox was used as a MOCK vector.

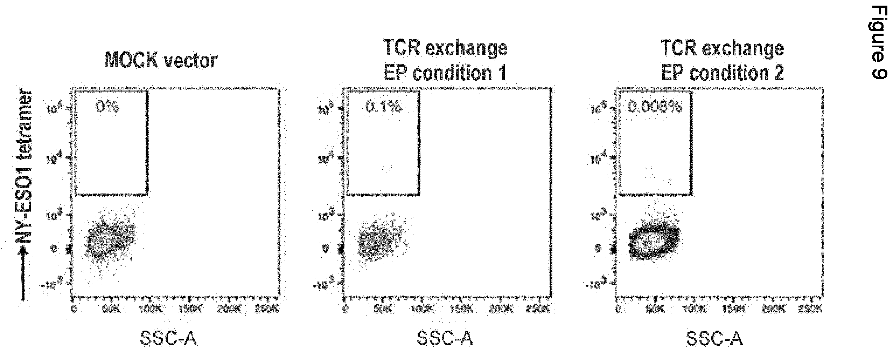

In order to exchange the WT1 specific TCR cassette tape gene in the cassette deck structure in the CD8 SP cells, the TCR cassette tape exchange vector or the MOCK vector together with the Cre expression vector (cCAG-nls-Cre) were introduced into the CD8 SP cells having the cassette deck structure with the WT1 specific TCR cassette tape by electroporation. The cells were cultured for 13 days after the electroporation. In order to confirm whether the TCR was exchanged, the presence of the NY-ESO1 tetramer positive cells were confirmed by FACS. Cells in which the WT1 specific TCR cassette tape was exchanged with the NY-ESO1 specific TCR cassette tape were detected as NY-ESO1 positive cells. Results are shown in Fig. 9. As a negative control, 10 µg of the MOCK vector that can express the GFP gene flanked by Lox2272 and Lox was used.

When the MOCK vector was used, NY-ESO1 tetramer positive cell was not generated at all. The inventors conducted the cassette tape exchange step twice with different conditions. NY-ESO1 tetramer positive cells were generated and confirmed that the cassette tape gene was successfully exchanged under each of the conditions.

### EXAMPLE 4

The marker protein, TCR was exchanged with a different TCR by means of genome editing.

The TCR cassette deck KI-Jurkat cells generated in Example 1 by knocking-in the rearranged-WT1 specific TCR gene in the Dβ2 region on the TCRβ gene locus of the cells were used. The TCRβ gene locus of the cells have the structure shown in Fig. 5A "WT1-TCR KI Human TCR locus". In this example 4, those cells are indicated as "Jurkat WT1-TCR cells". In this example, the WT1-TCR in the Jurkat WT1-TCR cells was exchanged with the NYESO1-TCR by using the genome editing.

Two types of guide RNAs (gRNA #1 and gRNA#4) targeting the sequence encoding the CDR3 region on the TCRβ chain were prepared. The 5' arm and 3' arm were determined to include the sequences from the cleavage site of each guide RNA to 20bp upstream and to 20bp downstream, respectively. The schematic diagram of the design for the homologous sequences of the two guide RNAs and the 5' and 3' arm is shown in Fig. 10. In the figure, the vertical lines separating the sequences correspond to the reading frame for translation into protein. Another guide RNA (gRNA#5) for the cleavage of the NYESO-1-TCR KI vector was also used. gRNA#5 recognized the underlined part of the sequence below:
5'-GCATCGTACGCGTACGTGTTTGG-3' (SEQ ID NO: 15)

A DNA sequence that had previously been reported was used for the sequence of gRNAregion of gRNA#5 vector (Nature Protocol 11, 118-133, 2016).

A chimeric gene comprising a chimeric TCRβ chain and a chimeric TCRα chain linked by a gene encoding p2A peptide was used as NYESO1-TCR gene to be exchanged with the WT1-TCR gene (Fig. 11). The chimeric TCRβ chain comprises the variable region (Vβ) of a known human TCR that recognizes NYESO1 antigen and a mouse TCR constant region (mCβ) which is linked to the Vβ, and the chimeric TCRα chain was prepared in the same manner as the chimeric TCRβ chain.

The schematic diagram of NYESO1-TCR gene KI vector is shown in Fig. 11. NYESO1-TCR KI vector is a plasmid DNA vector comprising, in order from the upstream to downstream, gRNA#5 recognition sequence, 5' arm sequence, p2A sequence, NYESO1-TCR gene in which TCRβ chain TCRα chain were linked by p2A sequence, 3' arm sequence, and gRNA#5 recognition sequence. The stop codon is included at the 3' end of NYESO1-TCR gene.

Preparation of guide RNA for CRISPR/Cas9 expression vector.

Vectors for CRISPR/Cas9 were prepared by using pX330 which can co-express guide RNA and Cas9. CRISPR/Cas9 vector comprising gRNA#1, gRNA#4 or gRNA#5, or a combination of gRNA#1 and gRNA#5, or a combination of gRNA# 4 and gRNA#5 was prepared. A schematic diagram of a vector for the expression of gRNA# 1, gRNA#5 and Cas9 is shown in Fig. 12 as one example.

Introduction of NYESO1-TCR KI vector and CRISPR/Cas9 vector in the Jurkat WT-TCR cells.

The vectors were introduced into the cells by electroporation. Electroporation was performed according to the manual of the Amaxa^{®} Cell Line Nucleofector^{®} Kit V . In 100µL of the reagent provided in the kit, 2 × 10⁶ cells and the vector were suspended. The suspension was transferred to the cuvette provided in the kit, the cuvette was set in the Amaxa Nucleofector II (Lonza), and the vectors were introduced into the cells by the built-in program X001.

### Conditions for electroporation

### Condition 1: gRNA#1 was used for cleaving WT1-TCR gene

**[Table 8]**

| 1-1 | | |
|---|---|---|
| DNA1 | NYESO1-TCR KI vector_#1 | 1 µg |
| DNA2 | CRISPR/Cas9(gRNA# 1 &5)vector | 1 µg |

| 1-2 | | |
|---|---|---|
| DNA1 | NYESO1-TCR KI vector_#1 | 2 µg |
| DNA2 | CRISPR/Cas9(gRNA#1&5) vector | 2 µg |

| 1-3 | | |
|---|---|---|
| DNA1 | NYESO1-TCR KI vector_#1 | 1 µg |
| DNA2 | CRISPR/Cas9(gRNA#1) vector | 0.5 µg |
| DNA3 | CRISPR/Cas9(gRNA#5) vector | 0.5 µg |

| 1-4 | | |
|---|---|---|
| DNA1 | NYESO1-TCR KI vector_#1 | 2 µg |
| DNA2 | CRISPR/Cas9(gRNA#1) vector | 1 µg |
| DNA3 | CRISPR/Cas9(gRNA#5) vector | 1 µg |

### Condition 2: gRNA#4 was used for cleaving WT1-TCR gene

**[Table 9]**

| 2-1 | | |
|---|---|---|
| DNA1 | NYESO1-TCR KI vector_#4 | 1 µg |
| DNA2 | CRISPR/Cas9(gRNA#4&5)vector | 1 µg |

| 2-2 | | |
|---|---|---|
| DNA1 | NYESO1-TCR KI vector_#4 | 2 µg |
| DNA2 | CRISPR/Cas9(gRNA#4&5) vector | 2 µg |

| 2-3 | | |
|---|---|---|
| DNA1 | NYESO1-TCR KI vector_#4 | 1 µg |
| DNA2 | CRISPR/Cas9(gRNA#4) vector | 0.5 µg |
| DNA3 | CRISPR/Cas9(gRNA#5) vector | 0.5 µg |

| 2-4 | | |
|---|---|---|
| DNA1 | NYESO1-TCR KI vector _#4 | 2 µg |
| DNA2 | CRISPR/Cas9(gRNA#4) vector | 1 µg |
| DNA3 | CRISPR/Cas9(gRNA#5) vector | 1 µg |

On 6 days after the introduction of the genes, expression of the TCR in the 8 types cells obtained by the conditions 1-1 to 2-4 as well as in the Jurkat WT1-TCR cells that were not subjected to the electroporation were analyzed by flow cytometry. The two antibodies and one tetramer shown below were used for the analysis.
Antibodies
   Human TCRαβ antibody-PECy7(BioLegend, 306720)
   Mouse TCRβ antibody-APC(BioLegend, 109212)
Tetramer
   NYESO1-tetramer-PE(MBL, TB-M011-1)

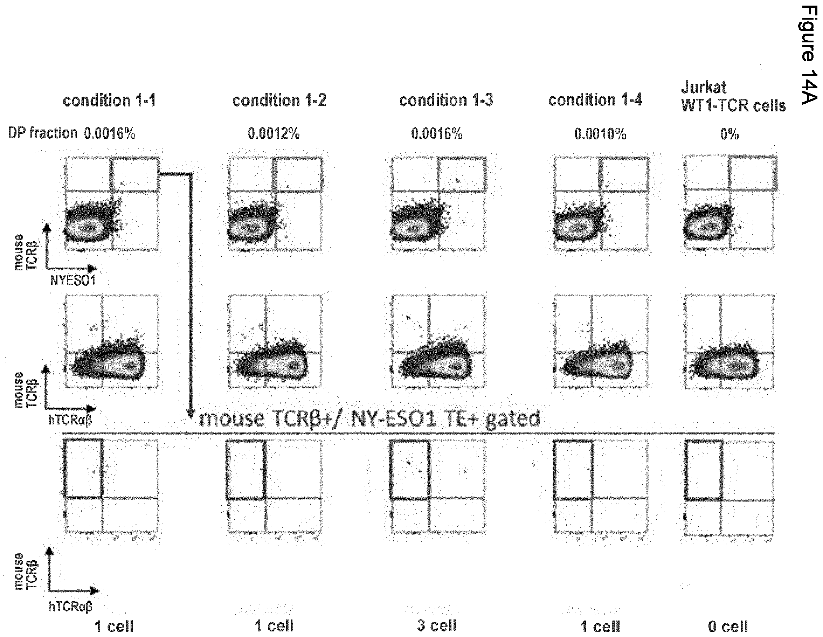

The human TCRαβ antibody recognizes TCRs having a human C region but not TCRs having a mouse C region. On the other hand, mouse TCRβ antibody recognizes TCRs having a mouse C region but not TCRs having a human C region. NYESO1 tetramer recognizes only NYESO1-TCR. In this example, human TCRαβ antibody was considered to recognize WT1-TCR (KM#3-3), and mouse TCRβ antibody as well as NYESO1-tetramer were considered to recognize NYESO1-TCR. Results are shown in Figs. 14A and 14B.

The Jurkat WT1-TCR cells into which no gene was introduced were confirmed to express TCR(KM#3-3) recognized by the human TCRαβ antibody but no cells whose TCR was recognized by the mouse TCRβ antibody or NYESO1-tetramer was confirmed (Fig. 14A and B, in the panels indicated as Jurkat WT1-TCR). In the cells obtained by knocking-in the NYESO1-TCR KI vector together with the gRNA/Cas9 vector for knocking-in the NYESO1-TCR at the CD3 region of the WT1-TCR, a cell population expressing NYESO1-TCR, although the percentage was very low. In addition, almost all cells expressing NYESO1-TCR did not express WT1-TCR (Fig. 14A, the bottom panels of conditions 1-1, 1-2, 1-3 and 1-4, Fig. 14B, the bottom panels of conditions 2-1, 2-2 and 2-4). According to the results, in was concluded that the TCR expressed in the Jurkat WT1-TCR cells were exchanged from WT1-TCR with NYESO1-TCR. This example confirms that the TCR expressed in the T cells can be exchanged with another TCR by genome editing.

## Claims

1. A method for producing effector cells which express a desired protein, comprising the steps of:
(1) providing material cells that have a cassette deck structure with a cassette tape gene comprising a gene encoding a marker protein in their genome, wherein the material cells can be differentiated into the effector cells, and wherein the marker protein can be expressed in the effector cells or progenitor cells thereof when the material cells are differentiated into the effector cells or progenitor cells thereof;
(2) proliferating the material cells,
(3) differentiating the material cells into the effector cells or progenitor cells thereof; and
(4) replacing the gene encoding the marker protein in the effector cells or progenitor cells thereof with a gene encoding the desired protein.

2. The method according to claim 1, wherein the upstream and downstream of the gene encoding the marker protein in the material cell in step (1) are flanked by a pair of recombinase target sequences, and wherein the step (4) is the step of culturing the effector cells or progenitor cells thereof with a cassette tape exchange vector comprising the gene encoding the desired protein flanked by the same recombinase target sequences in the presence of the recombinase so that the gene encoding the marker protein is exchanged with the gene encoding the desired protein.

3. The method according to claim 1, wherein the step (4) is the step of introducing the gene encoding the desired protein into the effector cells or progenitor cells thereof by means of genome editing so that the desired protein is expressed in the effector cells or the progenitor cells thereof instead of the marker protein.

4. The method according to any one of claims 1 to 3, further comprising the step of selecting the effector cells or progenitor cells thereof that are negative for the marker protein so that the effector cells or progenitor cells expressing the desired protein are selected.

5. The method according to any one of claims 1 to 4, wherein the marker protein is a known ligand or receptor that is expressed on the surface of the effector cells or the progenitor cells thereof.

6. The method according to claim 5, wherein the gene encoding the marker protein is a gene encoding a known rearranged T cell receptor (TCR) or a chimeric antigen receptor (CAR).

7. The method according to any one of claims 1-4, wherein the marker protein is a fluorescent protein.

8. The method according to any one of claims 1 to 7, wherein the material cells having the cassette deck structure provided in step (1) comprise only one cassette deck structure in the genome per one material cell.

9. The method according to claim 8, wherein in step (4), the cassette tape gene in the cassette deck structure contained in the clonal effector cells or progenitor cells thereof is exchanged with multiple cassette tape genes each comprising a gene encoding different desired protein so that effector cells expressing different proteins are produced at the same time.

10. The method according to claim 9, wherein the cassette tape gene comprises a pair of recombinase target sequences in its upstream and downstream, and wherein in step (4), the effector cells or progenitor cells thereof are cultured with multiple cassette tape exchange vectors at the same time in the presence of the recombinase.

11. The method according to claim 9, wherein in step (4), the cassette tape gene in the effector cells or progenitor cells thereof is exchanged with multiple cassette tape genes each comprising a gene encoding different desired protein by means of genome editing.

12. The method according to any one of claims 1 to 11, wherein the material cell is a pluripotent stem cell.

13. The method according to claim 12, wherein the pluripotent stem cell is an ES cell or an iPS cell.

14. A method for producing multiple types of effector cells each expressing different protein, comprising the steps of:
providing clonal effector cells or progenitor cells thereof which comprises in its genome one cassette deck structure per one cell, wherein the cassette deck structure comprises a cassette tape gene comprising a gene encoding a marker protein in the manner the marker protein can be expressed in the cells and does not comprise exogenous drug resistant gene, and
exchanging the gene encoding the marker protein in the effector cells or progenitor cells thereof with genes encoding multiple proteins to produce multiple types of effector cells each expressing different protein at the same time.

15. The method according to claim 14, wherein the cassette tape gene comprises a pair of recombinase target sequences in its upstream and downstream, and the effector cells or progenitor cells thereof comprise the cassette deck structure with a cassette tape gene comprising the gene encoding the marker protein are cultured with multiple cassette tape exchange vectors each comprising a gene encoding different protein under the presence of the recombinase to produce multiple types of effector cells each expressing different protein at the same time.

16. The method according to claim 15, wherein the step of exchanging the gene encoding the marker protein in the effector cells or progenitor cells thereof with genes encoding multiple desired proteins is carried by means of genome editing.

17. The method according to any one of claims 1 to 16, wherein the effector cell is an immune cell.

18. The method according to claim 17, wherein the effector cell is NK cell or T cell.

19. The method according to claim 17 or 18, wherein the, wherein the gene encoding the marker protein is a gene encoding a known rearranged T cell receptor (TCR) or a chimeric antigen receptor.

20. The method according to any one of claims 17 to 19, wherein the desired protein is TCR or CAR.

21. A composition comprising the effector cells obtained by the method of claim 20, wherein the composition is for the treatment of an immune relating disease in a subject.
